# EUROPEAN PATENT APPLICATION

(11) **EP 4 763 840 A1**
(43) Date of publication of application: **24.06.2026**
(21) Application number: 24863213.5
(22) Date of filing: 05.09.2024
(51) Int. Cl.: C07D 403/04, A61K 31/517, A61K 31/5377, A61P 35/00, C07D 401/14

(54) **NOVEL QUINAZOLINE-BENZIMIDAZOLE DERIVATIVE OR PHARMACEUTICALLY ACCEPTABLE SALT THEREOF AND USE THEREOF**

(30) Priority: 05.09.2023 KR 20230117582
(71) Applicant: Korea University Research and Business Foundation, Seoul 02841 (KR)
(72) Inventor: SEO, Jae Hong, Gwacheon-si Gyeonggi-do 13803 (KR); NAM, Kee Dal, Seoul 02467 (KR); KIM, Ji Young, Seoul 08226 (KR); KIM, Yoon Jae, Paju-si Gyeonggi-do 10887 (KR); JUNG, Eun Sun, Incheon 21122 (KR); KANG, Yong Koo, Seoul 04426 (KR); PARK, So-Eun, Daegu 42685 (KR); PARK, Min Su, Seoul 08298 (KR); KIM, Seong-Jae, Seoul 03493 (KR); LEE, Da Eun, Daegu 41424 (KR)
(74) Representative: Cousens, Nico
(86) International application number: PCT/KR2024/013396
(87) International publication number: WO 2025/053634

(57) **Abstract**

The present invention relates to a quinazoline-benzimidazole derivative or a pharmaceutically acceptable salt thereof and a composition comprising the derivative as an active ingredient for preventing or treating cancer. The quinazoline-benzimidazole derivative of the present invention is activated in cancer cells to inhibit tubulin polymerization and tyrosine kinase activity of the human epidermal growth factor receptor 2 (HER2). Upon administration to a subject, the derivative can arrest the cell cycle of cancer cells, induce apoptosis, and block cell growth signals transmitted via the HER2 receptor. Therefore, the derivative can be used as a dual-target anticancer drug for the prevention or treatment of cancer, particularly HER2-positive breast cancer or triple-negative breast cancer.

## Description

### TECHNICAL FIELD

The disclosure relates to a quinazoline-benzimidazole derivative or a pharmaceutically acceptable salt thereof, and a use thereof.

### BACKGROUND ART

Patients with triple-negative breast cancer (TNBC; ER-, PR-, HER2-) account for 10 to 15% of all breast cancer patients, and since hormone receptors (ER (estrogen receptor), PR (progesterone receptor)) and HER2 protein are absent, these patients do not benefit from hormone therapy or HER2-targeted therapeutics. Currently, the standard treatment for triple-negative breast cancer is entirely dependent on general cytotoxic anticancer agents (Taxene-based or Anthracycline-based). Because there is no established targeted therapeutic agent, treatment strategies for other subtypes are not as diverse compared to breast cancer. More seriously, after surgery or anticancer treatment, recurrence occurs within 2 to 3 years in most patients, and metastasis to other organs, such as the lungs, the liver, the brain, and the bones, is easily induced, which affects a decrease in patient survival rates.

The 5-year overall survival of patients diagnosed with stage III (stage-III) is 55% or less, and for patients with advanced-stage cancer in which cancer metastasis has already progressed, the 5-year overall survival is very low at 30% or less. Most of these patients ultimately die within several years, making it a very serious disease.

A microtubule is a major component of a cytoskeleton, and is composed of a tubulin heteropolymer consisting of an α subunit and a β subunit. Microtubules perform various cellular functions such as intracellular transport, polarity maintenance, intracellular signal transduction, cell migration, and proliferation. During mitosis of a cell, a spindle fiber is formed to perform a process in which chromosomes are aligned at the center of the cell and then separated toward both poles. When the spindle fiber fails to function properly, cell division is inhibited and apoptosis occurs, and thus, the spindle fiber has attracted attention as a target for anticancer agents.

Drugs targeting microtubules are largely divided into two groups: drugs that stabilize microtubules and drugs that destabilize microtubules. First, microtubule stabilizers include Taxane, Paclitaxel (Taxol), and Decetaxel, and these stabilizers function to prevent depolymerization of microtubules and to enhance polymerization. Most microtubule-stabilizing substances bind to a Taxane-binding site or an overlapping site of β-tubulin. Second, microtubule destabilizers include colchicine and vinca alkaloid, and these destabilizers bind to a colchicine binding site or a vinca binding site. Drugs targeting the microtubules themselves exhibit effects at lower concentrations than drugs affecting microtubule polymers, and the point that these drugs ultimately inhibit cell mitosis is the same. Therefore, there is a need to develop potential tubulin polymerization inhibitors as anticancer agents.

The HER2 (Human Epidermal Growth Factor Receptor 2) receptor is known as one of the major factors that is overexpressed in various cancers, particularly breast cancer and gastric cancer, and promotes growth, survival, differentiation, and metastasis of cancer cells. Activation of HER2 activates intracellular signal transduction pathways through a tyrosine kinase domain of the receptor, and these pathways mainly include a RAS/RAF/MEK/ERK pathway and a PI3K/AKT/mTOR pathway. These signal transduction pathways induce rapid proliferation and anti-apoptosis (inhibition of cell death) of cancer cells, thereby promoting the formation and progression of a tumor.

In previous studies, various compounds that are capable of blocking these signal transduction pathways and effectively inhibiting growth and metastasis of cancer cells by inhibiting tyrosine kinase activity of HER2 have been developed. Representative HER2 tyrosine kinase inhibitors include lapatinib and neratinib, and these drugs inhibit signal transduction by binding to an ATP-binding site of the HER2 receptor and blocking autophosphorylation of tyrosine residues. These drugs are used as effective therapeutic agents, particularly in patients with HER2-positive breast cancer. However, the need for the development of new HER2 tyrosine kinase inhibitors has been continuously raised due to drug resistance and side effects.

### DISCLOSURE OF THE INVENTION

### TECHNICAL GOALS

A technical problem to be achieved by the disclosure is to provide a quinazoline-benzimidazole derivative or a pharmaceutically acceptable salt thereof.

In addition, another object of the disclosure is to provide a pharmaceutical composition for preventing or treating cancer, including, as an active ingredient, the quinazoline-benzimidazole derivative or the pharmaceutically acceptable salt thereof.

Still another object of the disclosure is to provide a method of preparing the quinazoline-benzimidazole derivative or the pharmaceutically acceptable salt thereof.

However, technical goals to be achieved are not limited to those described above, and other goals not mentioned above are clearly understood by one of ordinary skill in the art from the following description.

### TECHNICAL SOLUTIONS

In order to solve the above problem, the disclosure provides a quinazoline-benzimidazole derivative represented by [Formula 1], or a pharmaceutically acceptable salt thereof:

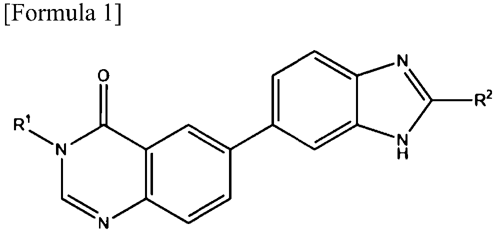

in Formula 1,
R¹ may be -(CH₂)ₙ-A,
A may be any one of a substituted or unsubstituted C₃-C₂₀ cycloalkyl and a C₃-C₂₀ heterocycloalkyl,
when the cycloalkyl or the heterocycloalkyl is substituted, the cycloalkyl or the heterocycloalkyl may be substituted with one or more selected from the group consisting of a C₁-C₆ alkyl group, a hydroxy group, an amine group, a methoxy group, and a halogen group,
R ² may be any one selected from the group consisting of a substituted or unsubstituted C₃-C₂₀ aryl, a substituted or unsubstituted C₃-C₂₀ heteroaryl, a substituted or unsubstituted C₃-C₂₀ cycloalkyl, a substituted or unsubstituted C₃-C₂₀ heterocycloalkyl, and a methyl carbamate,
when the aryl, the cycloalkyl, or the heterocycloalkyl is substituted, the aryl, the cycloalkyl, or the heterocycloalkyl may be substituted with one or more selected from the group consisting of a C₁-C₆ alkyl group, a halogen group, a hydroxy group, and an alkoxy group, and n may be an integer of 0 to 6.

According to an aspect,
in [Formula 1],
A may be a substituted or unsubstituted C₃-C₂₀ heterocycloalkyl,
when the heterocycloalkyl is substituted, the heterocycloalkyl may be substituted with one or more of a methyl group and a hydroxy group,
R₂ may be any one selected from the group consisting of a substituted or unsubstituted phenyl, a substituted or unsubstituted thiophenyl, a substituted or unsubstituted pyridinyl, a substituted or unsubstituted cyclopentyl, a substituted or unsubstituted cyclohexyl, and -NH-(C=O)-OCH₃,
when the phenyl, the thiophenyl, the pyridinyl, the cyclopentyl, and the cyclohexyl are substituted, the phenyl, the thiophenyl, the pyridinyl, the cyclopentyl, and the cyclohexyl may be substituted with one or more selected from the group consisting of methyl, methoxy, halogen, and hydroxy, and n may be 2.

According to an aspect,
in [Formula 1],
the C₃-C₂₀ heterocycloalkyl of A may be any one of morpholine and piperidine.

According to an aspect,
the quinazoline-benzimidazole derivative represented by [Formula 1] may be any one selected from the group consisting of the following compounds:

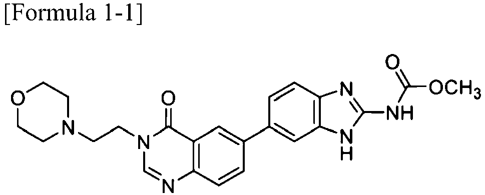

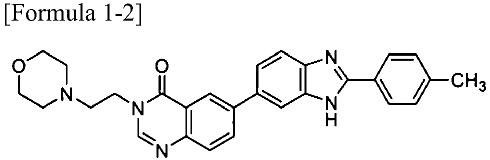

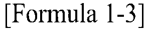

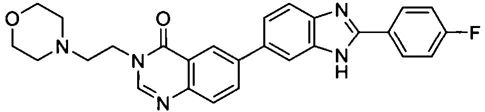

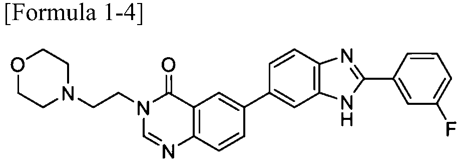

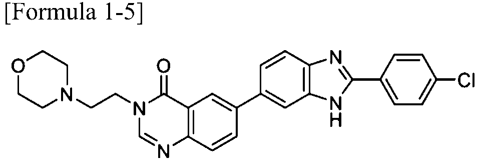

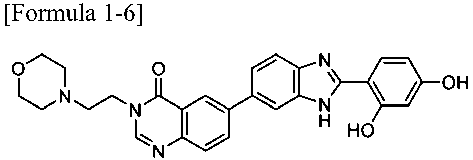

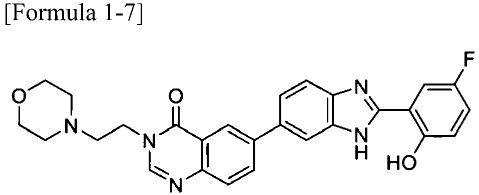

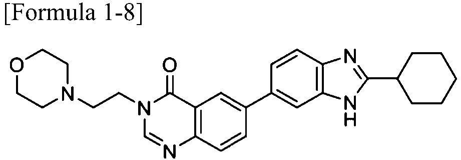

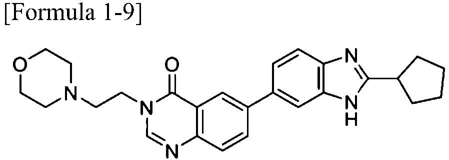

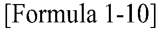

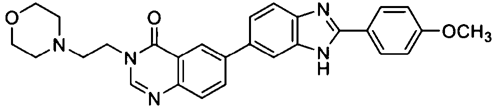

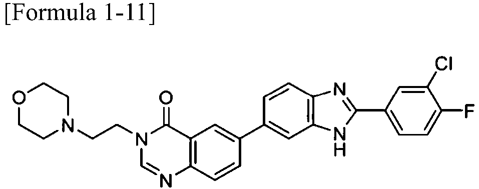

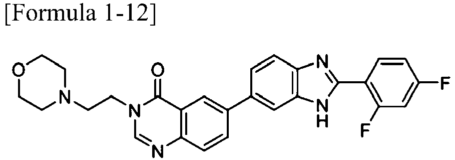

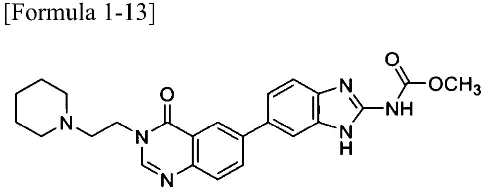

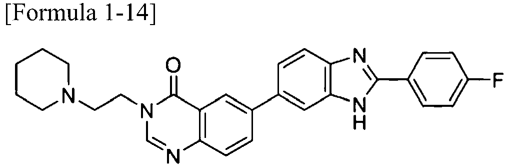

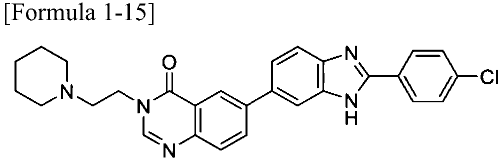

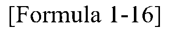

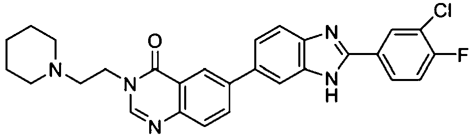

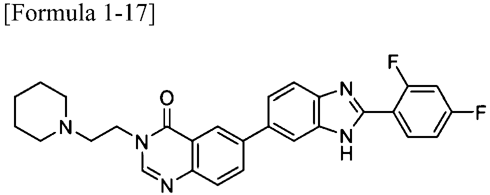

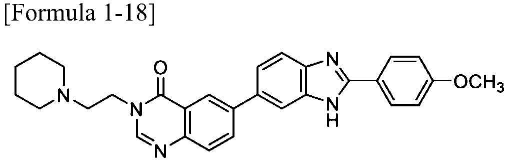

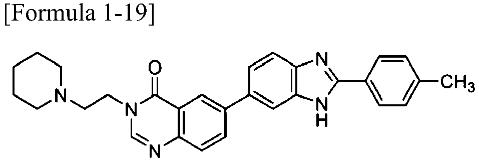

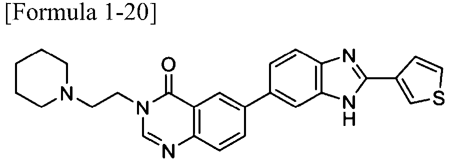

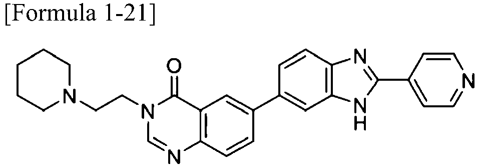

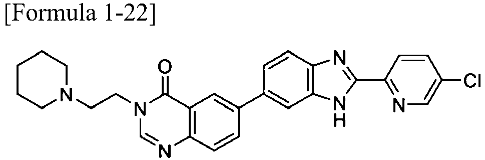

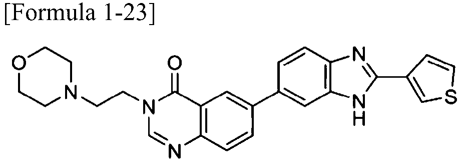

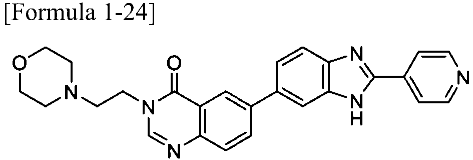

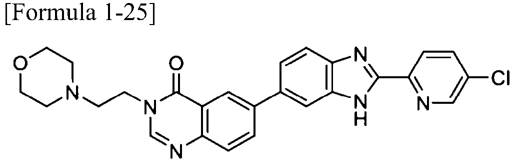

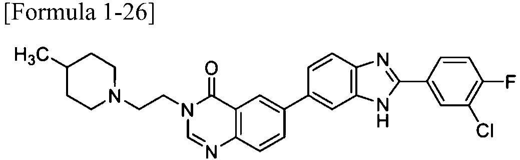

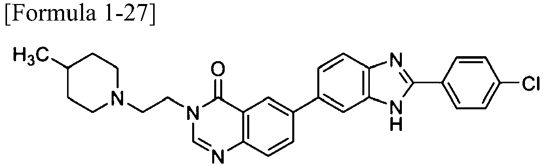

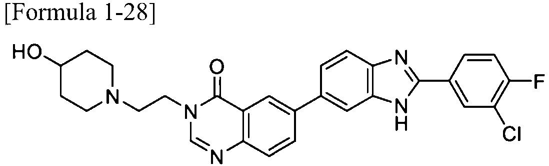

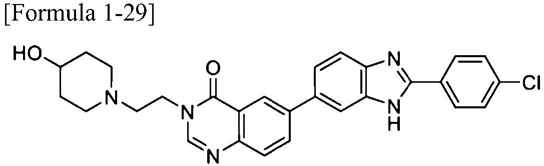

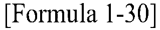

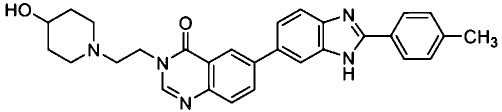

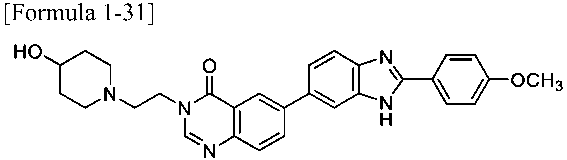

According to an aspect, the quinazoline-benzimidazole derivative may have both an effect of inhibiting tubulin polymerization and an effect of inhibiting a tyrosine kinase of human epidermal growth factor receptor 2 (HER2).

According to an aspect, the pharmaceutically acceptable salt of the quinazoline-benzimidazole derivative may be one or more selected from the group consisting of a hydrochloride, a bromate, a sulfate, a phosphate, a nitrate, a citrate, an acetate, a lactate, a tartrate, a maleate, a gluconate, a succinate, a formate, a trifluoroacetate, an oxalate, a fumarate, a glutarate, an adipate, a methanesulfonate, a benzenesulfonate, a para-toluenesulfonate, a camphorsulfonate, a sodium salt, a potassium salt, a lithium salt, a calcium salt, and a magnesium salt.

According to another embodiment of the disclosure, a pharmaceutical composition for preventing or treating cancer is provided, including, as an active ingredient, the quinazoline-benzimidazole derivative, or a pharmaceutically acceptable salt thereof.

According to an aspect, the pharmaceutical composition may induce apoptosis by causing cell cycle arrest in cancer cells.

According to an aspect, the pharmaceutical composition may inhibit an activity of one or more of HER2, p95HER2, HER3, EGFR, AKT, and MEK.

According to an aspect, the cancer may be one or more selected from the group consisting of skin cancer, breast cancer, uterine cancer, esophageal cancer, gastric cancer, brain tumor, colon cancer, rectal cancer, colorectal cancer, lung cancer, ovarian cancer, cervical cancer, endometrial cancer, vulvar cancer, renal cancer, hematological cancer, pancreatic cancer, prostate cancer, testicular cancer, laryngeal cancer, head and neck cancer, thyroid cancer, hepatic cancer, bladder cancer, osteosarcoma, lymphoma, hematological cancer, thymic cancer, urethral cancer, and bronchial cancer, and preferably may be HER2-positive breast cancer or triple-negative breast cancer.

According to still another embodiment of the disclosure, there is provided a method of preparing a quinazoline-benzimidazole derivative represented by [Formula 1], or a pharmaceutically acceptable salt thereof, the method including the following preparation steps:
1) preparing a compound represented by [Formula 4] by reacting a compound represented by R¹-NH₂ with 5-bromoanthranilic acid and triethyl orthoformate;
2) preparing a compound represented by [Formula 2] by reacting the compound represented by [Formula 4] with any one of 2-nitro-4-(4,4,5,5-tetramethyl-1,3,2-dioxaborolan-2-yl)aniline and 4-amino-3-nitrophenylboronic acid pinacol ester;
3) preparing a compound represented by [Formula 3] from the compound represented by [Formula 2]; and
4) preparing a compound represented by [Formula 1] by reacting the compound represented by [Formula 3] with any one of 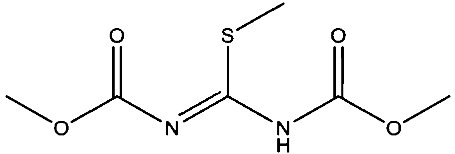 and a compound represented by R²-CHO; 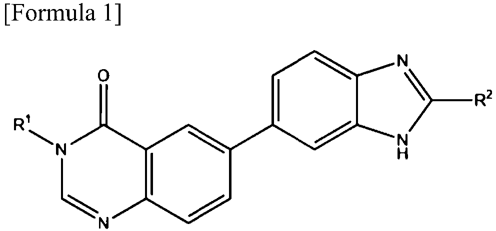 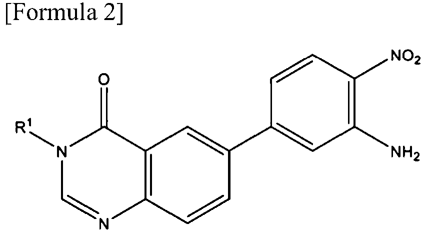 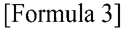 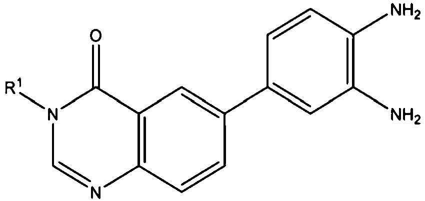 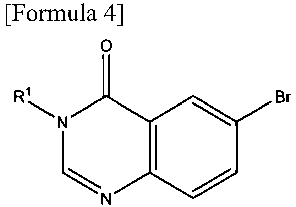 in Formulas 1 to 4,
   R¹ may be -(CH₂)ₙ-A,
   A may be any one of a substituted or unsubstituted C₃-C₂₀ cycloalkyl and a C₃-C₂₀ heterocycloalkyl,
   when the cycloalkyl or the heterocycloalkyl is substituted, the cycloalkyl or the heterocycloalkyl may be substituted with one or more selected from the group consisting of a C₁-C₆ alkyl group, a hydroxy group, an amine group, a methoxy group, and a halogen group,
   R² may be any one selected from the group consisting of a substituted or unsubstituted C₃-C₂₀ aryl, a substituted or unsubstituted C₃-C₂₀ heteroaryl, a substituted or unsubstituted C₃-C₂₀ cycloalkyl, a substituted or unsubstituted C₃-C₂₀ heterocycloalkyl, and a methyl carbamate,
   when the aryl, the cycloalkyl, or the heterocycloalkyl is substituted, the aryl, the cycloalkyl, or the heterocycloalkyl may be substituted with one or more selected from the group consisting of a C₁-C₆ alkyl group, a halogen group, a hydroxy group, and an alkoxy group, and n may be an integer of 0 to 6.

In addition, the disclosure provides a composition for preventing or treating cancer, including, as an active ingredient, the quinazoline-benzimidazole derivative or a pharmaceutically acceptable salt thereof.

In addition, the disclosure provides a method of preventing or treating cancer, including administering the quinazoline-benzimidazole derivative or a pharmaceutically acceptable salt thereof to a subject.

In addition, the disclosure provides a use of the quinazoline-benzimidazole derivative or a pharmaceutically acceptable salt thereof for the manufacture of a medicament for preventing or treating cancer.

In addition, the disclosure provides a method of diagnosing cancer, including administering the quinazoline-benzimidazole derivative or a pharmaceutically acceptable salt thereof to a subject.

In addition, the disclosure provides a use of the quinazoline-benzimidazole derivative or a pharmaceutically acceptable salt thereof for the manufacture of a medicament for diagnosing cancer.

As an embodiment of the disclosure, the pharmaceutical composition may induce apoptosis by causing cell cycle arrest in cancer cells, or may be for inhibiting a tyrosine kinase of human epidermal growth factor receptor 2 (HER2) to block a cell growth signal transmitted through a HER2 receptor, and preferably may be for performing both mechanisms.

As another embodiment of the disclosure, the cancer may be one or more selected from the group consisting of skin cancer, breast cancer, uterine cancer, esophageal cancer, gastric cancer, brain tumor, colon cancer, rectal cancer, colorectal cancer, lung cancer, ovarian cancer, cervical cancer, endometrial cancer, vulvar cancer, renal cancer, hematological cancer, pancreatic cancer, prostate cancer, testicular cancer, laryngeal cancer, head and neck cancer, thyroid cancer, hepatic cancer, bladder cancer, osteosarcoma, lymphoma, leukemia, thymic cancer, urethral cancer, and bronchial cancer, and preferably may be breast cancer, and more preferably may be HER2-positive breast cancer or triple-negative breast cancer.

### EFFECTS OF THE INVENTION

The disclosure relates to a quinazoline-benzimidazole derivative or a pharmaceutically acceptable salt thereof and a composition for preventing or treating cancer including the derivative as an active ingredient, and the quinazoline-benzimidazole derivative of the disclosure is activated in cancer cells to inhibit tubulin polymerization and simultaneously inhibit a tyrosine kinase of human epidermal growth factor receptor 2 (HER2), so that when administered to a subject, the derivative blocks a cancer cell cycle, induces apoptosis, and blocks a cell growth signal transmitted through an HER2 receptor, thereby inhibiting cell proliferation and survival and simultaneously obtaining an anticancer effect. Therefore, the compound of the disclosure and a composition comprising the same may be used as a novel dual-target anticancer composition for preventing or treating cancer, and preferably for preventing or treating HER2-positive breast cancer or triple-negative breast cancer.

### BRIEF DESCRIPTION OF DRAWINGS

FIG. 1 shows the results of evaluating changes in cell viability through an MTS assay after treatment with quinazoline-benzimidazole derivative compounds (1-1 to 1-10) at different concentrations (0, 1, and 5 µM) in a triple-negative breast cancer cell line, MDA-MB-231, and a HER2-positive breast cancer cell line, JIMT-1.
FIG. 2 shows the results of evaluating changes in cell viability through an MTS assay after treatment with quinazoline-benzimidazole derivative compounds (1-11 to 1-20) at different concentrations (0, 1, and 5 µM) in a triple-negative breast cancer cell line, MDA-MB-231, and a HER2-positive breast cancer cell line, JIMT-1.
FIG. 3 shows the results of evaluating changes in cell viability through an MTS assay after treatment with quinazoline-benzimidazole derivative compounds (1-21 to 1-31) at different concentrations (0, 1, and 5 µM) in a triple-negative breast cancer cell line, MDA-MB-231, and a HER2-positive breast cancer cell line, JIMT-1.
FIG. 4 shows cell viability and cell death morphology in trastuzumab-sensitive and trastuzumab-resistant HER2-positive breast cancer cell lines.
FIG. 5 shows confirmation that tubulin is degraded and apoptosis is induced in SKBR3 and JIMT-1 cell lines.
FIG. 6 shows confirmation of G2/M phase cell cycle arrest and expression of a mitotic marker.
FIG. 7 shows confirmation of expression and activity of HER2, HER family members, and downstream signaling factors by western blot.
FIG. 8 shows confirmation of the effects of quinazoline-benzimidazole derivative compounds 1-16 and 1-19 in lung cancer, colorectal cancer, ovarian cancer, prostate cancer, hepatic cancer, and hematological cancer cell lines.

### BEST MODE FOR CARRYING OUT THE INVENTION

The present inventors have confirmed that a benzimidazole derivative has an anticancer effect by inhibiting tubulin synthesis, thereby causing cell cycle arrest and thereby inhibiting cancer cell division, and that a quinazoline derivative may have an anticancer effect by inhibiting a tyrosine kinase of human epidermal growth factor receptor 2 (HER2), and have confirmed the possibility of activating both mechanisms of action by combining benzimidazole and quinazoline within a single compound molecule, and thus, they have completed the invention and intend to provide a quinazoline-benzimidazole derivative or a pharmaceutically acceptable salt thereof as a novel dual-target anticancer drug.

From the above results, the disclosure provides a quinazoline-benzimidazole derivative represented by [Formula 1], or a pharmaceutically acceptable salt thereof:

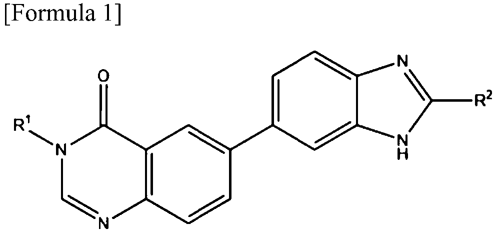

In Formula 1,
R1 is -(CH2)n-A,
A is any one of a substituted or unsubstituted C3-C20 cycloalkyl and a C3-C20 heterocycloalkyl,
when the cycloalkyl or the heterocycloalkyl is substituted, the cycloalkyl or the heterocycloalkyl is substituted with one or more selected from the group consisting of a C1-C6 alkyl group, a hydroxy group, an amine group, a methoxy group, and a halogen group,
R2 is any one selected from the group consisting of a substituted or unsubstituted C3-C20 aryl, a substituted or unsubstituted C3-C20 heteroaryl, a substituted or unsubstituted C3-C20 cycloalkyl, a substituted or unsubstituted C3-C20 heterocycloalkyl, and a methyl carbamate,
when the aryl, the cycloalkyl, or the heterocycloalkyl is substituted, the aryl, the cycloalkyl, or the heterocycloalkyl is substituted with one or more selected from the group consisting of a C₁-C₆ alkyl group, a halogen group, a hydroxy group, and an alkoxy group, and n is an integer of 0 to 6.

In the disclosure, the term "substitution" refers to a reaction in which an atom or atomic group included in a molecule of a compound is replaced with another atom or atomic group.

In the disclosure, the term "chain" refers to a molecule having a chain structure, and the chain structure is a chemical structure in which carbon atoms are linked in a chain shape, and there are straight chain shapes and branched shapes.

In the disclosure, the term "cyclic" refers to a structure in which both ends of the skeleton of an organic compound are connected to form a ring shape.

In the disclosure, the term "linear or branched alkyl group" means a monovalent linear or branched hydrocarbon residue consisting only of carbon and hydrogen atoms, having 1 to 6 carbon atoms.

In the disclosure, the term "cyclic alkyl group" or "cycloalkyl group" means a cyclic saturated hydrocarbon residue.

Examples of the alkyl groups include, but are not limited to, methyl, ethyl, propyl, isopropyl, butyl, isobutyl, 2-butyl, 3-butyl, pentyl, n-hexyl, cyclobutyl, cyclopentyl, cyclohexyl, and the like.

In the disclosure, the term "heterocycloalkyl group" typically refers to a saturated or unsaturated (but not aromatic) cyclohydrocarbon, which may be optionally unsubstituted, monosubstituted or polysubstituted, and in the structure of which at least one is selected from a heteroatom of N, O or S.

In the disclosure, the term "aryl group" means an unsaturated aromatic ring compound having 3 to 12 carbon atoms having a single ring (e.g., phenyl) or multiple condensed rings (e.g., naphthyl). Examples of such aryl groups include, but are not limited to, phenyl, naphthyl, and the like.

In the disclosure, the term "heteroaryl group" refers to a single ring or multiple condensed rings in which at least one of the atoms constituting the ring has a heteroatom of N, O or S. Examples of such heteroaryl groups include, but are not limited to, a pyridyl group, a pyrimidinyl group, a pyrazinyl group, an oxazolyl group, and a furyl group.

In the disclosure, the term "alkoxy group" means an alkyl group (-O-R) bonded to oxygen. Examples of such alkoxy groups include, but are not limited to, methoxy groups, ethoxy groups, propoxy groups, and butoxy groups.

In the disclosure, the "halogen group" may be fluorine (F), chloride (Cl), bromine (Br), or iodine (I).

As a preferred embodiment of the disclosure, the compound represented by Formula 1 is preferably at least one selected from the group consisting of the following compounds:

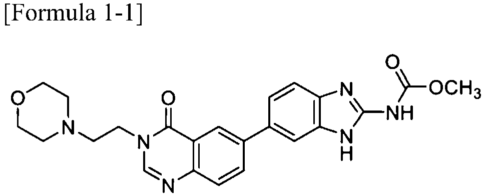

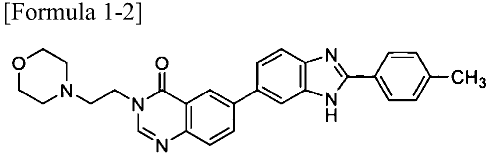

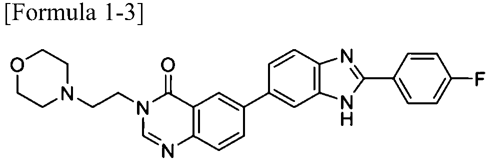

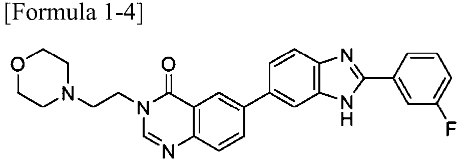

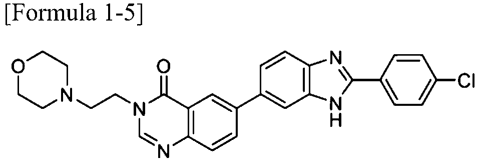

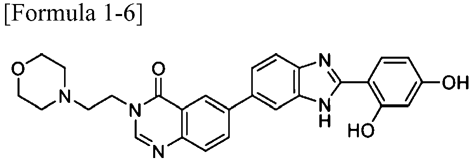

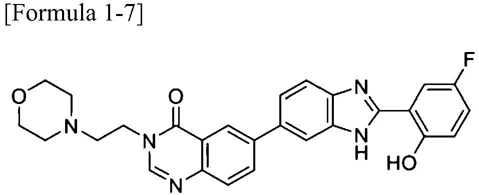

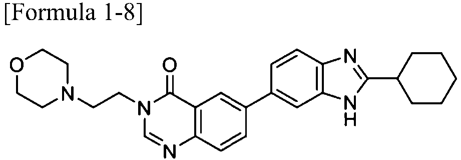

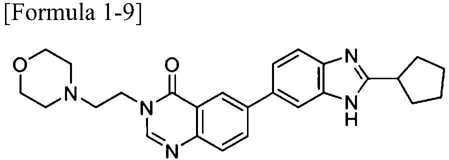

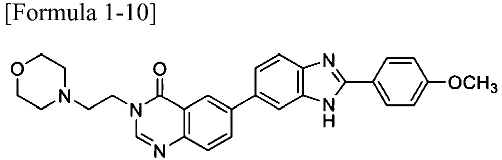

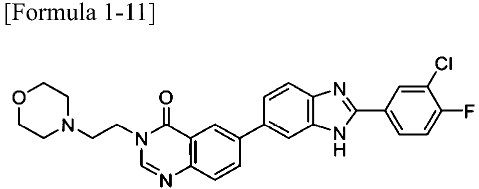

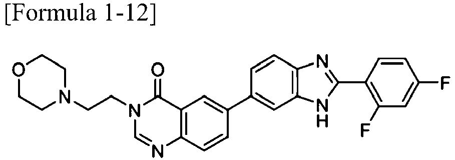

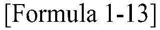

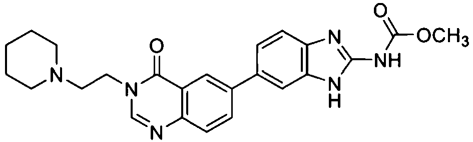

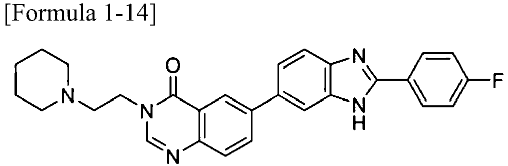

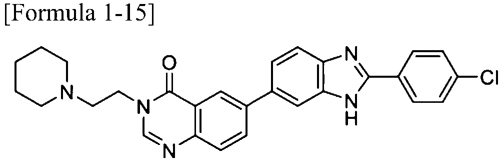

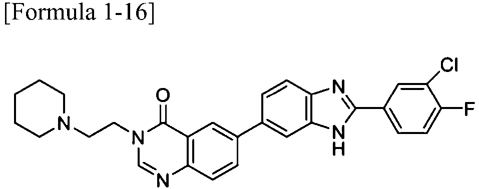

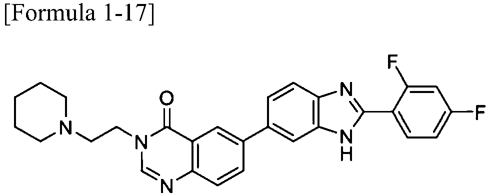

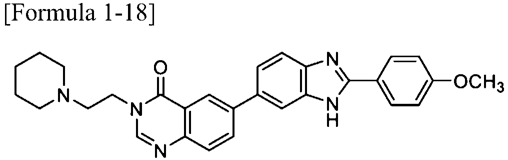

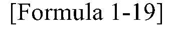

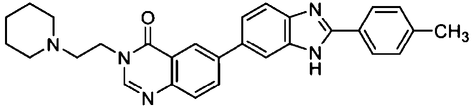

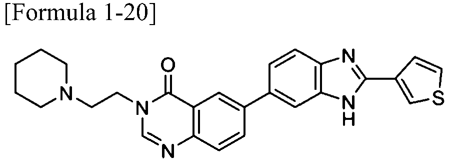

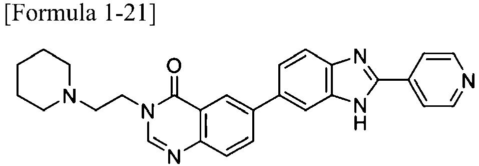

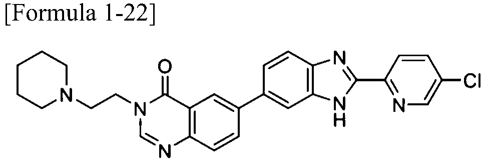

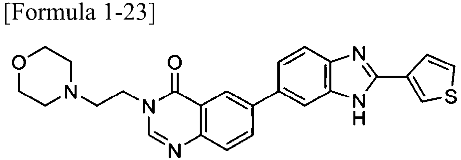

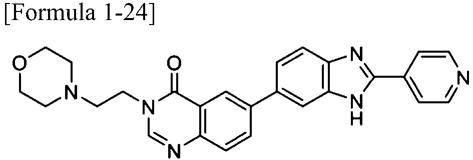

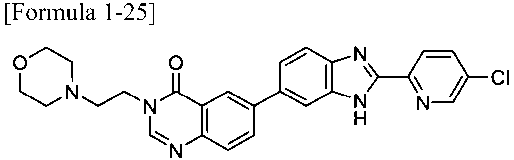

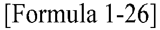

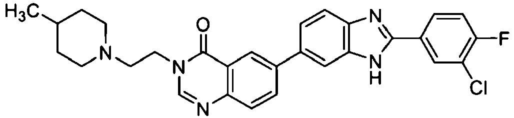

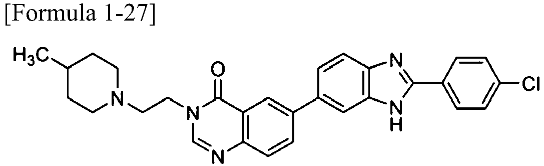

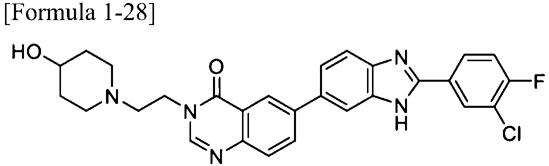

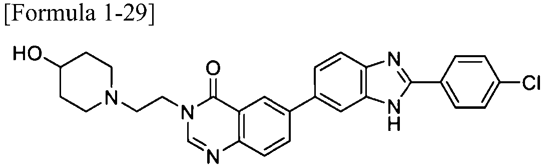

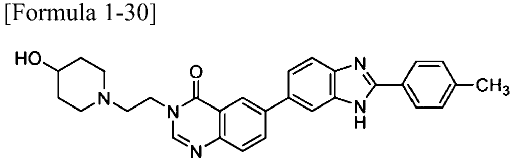

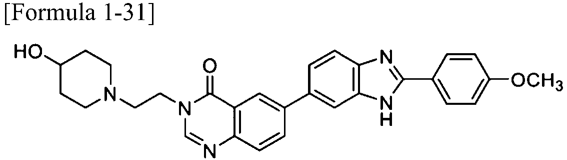

The quinazoline-benzimidazole derivative or the pharmaceutically acceptable salt thereof of the disclosure is activated specifically in cancer cells to inhibit tubulin polymerization and induce apoptosis, and is capable of inhibiting a tyrosine kinase of human epidermal growth factor receptor 2 (HER2) to block a cell growth signal transmitted through an HER2 receptor and inhibiting activities of HER2, p95HER2, HER3, EGFR, AKT, and MEK, and thus the quinazoline-benzimidazole derivative or the pharmaceutically acceptable salt thereof may be used as a pharmaceutical composition for preventing or treating cancer including the same as an active ingredient.

As an embodiment of the disclosure, the pharmaceutical composition may induce apoptosis by causing cell cycle arrest in cancer cells, or may block a cell growth signal transmitted through an HER2 receptor, and preferably may perform both mechanisms.

In the disclosure, the term "pharmaceutically acceptable salt" means a formulation of a compound that does not cause serious irritation to an organism to which the compound is administered and does not impair the biological activity and physical properties of the compound. The pharmaceutically acceptable salt may be obtained by reacting the compound of the disclosure with an inorganic acid such as hydrochloric acid, bromic acid, sulfuric acid, nitric acid, or phosphoric acid; a sulfonic acid such as methanesulfonic acid, ethanesulfonic acid, or p-toluenesulfonic acid; or an organic carboxylic acid such as tartaric acid, formic acid, citric acid, acetic acid, trichloroacetic acid, trifluoroacetic acid, capric acid, isobutanoic acid, malonic acid, succinic acid, phthalic acid, gluconic acid, benzoic acid, lactic acid, fumaric acid, maleic acid, or salicylic acid. In addition, the pharmaceutically acceptable salt may be obtained by reacting the compound of the disclosure with a base to form a salt, including salts such as ammonium salts; alkali metal salts such as sodium or potassium salts; alkaline earth metal salts such as calcium or magnesium salts; salts of organic bases such as dicyclohexylamine, N-methyl-D-glucamine, or tris(hydroxymethyl)methylamine; and amino acid salts such as arginine or lysine.

Furthermore, the quinazoline-benzimidazole derivative or the pharmaceutically acceptable salt thereof may include not only pharmaceutically acceptable salts but also all salts, hydrates, and solvates capable of being prepared by conventional methods.

In addition, the disclosure may provide a method of preventing, treating, and/or diagnosing cancer, including administering the quinazoline-benzimidazole derivative or a pharmaceutically acceptable salt thereof to a subject.

In the disclosure, the term "prevention" means any act of inhibiting or delaying the occurrence, spread or recurrence of the cancer by administering the composition of the disclosure, and "treatment" means any act of improving or beneficially changing the symptoms of the disease by administering the composition of the disclosure.

In the disclosure, the term "pharmaceutical composition" means a composition manufactured for the purpose of preventing or treating the above-described disease, and can be formulated and used in various forms according to conventional methods. For example, the pharmaceutical composition can be formulated into oral dosage forms such as powder, granules, tablets, capsules, suspensions, emulsions, and syrups, and can be formulated and used in the form of external preparations, suppositories, and sterile injectable solutions.

In the disclosure, "including as an active ingredient" means that the corresponding component is included in an amount necessary or sufficient to realize a desired biological effect. In actual application, the determination of the amount included as an active ingredient is an amount for treating a target disease, and may be determined by considering factors that do not cause other toxicity, and may vary according to various factors such as, for example, the disease or condition being treated, the form of the composition being administered, the size of the subject, or the severity of the disease or condition. A person skilled in the art to which the disclosure pertains can empirically determine the effective amount of an individual composition without undue experimentation.

In addition, the pharmaceutical composition of the disclosure may, depending on each formulation, additionally contain one or more pharmaceutically acceptable carriers in addition to the active ingredients described above.

The pharmaceutically acceptable carrier may be saline solution, sterile water, Ringer's solution, buffered saline, dextrose solution, maltodextrin solution, glycerol, ethanol, and mixtures of one or more of these components, and may further include other conventional additives such as antioxidants, buffers, and bacteriostatic agents, as needed. In addition, diluents, dispersants, surfactants, binders and lubricants may be additionally added to formulate the composition into injectable formulations such as aqueous solutions, suspensions and emulsions, pills, capsules, granules or tablets. Furthermore, the composition may be preferably formulated for each disease or ingredient using an appropriate method in the art or a method disclosed in Remington's Pharmaceutical Science (Mack Publishing Company, Easton PA).

The composition of the disclosure may be administered orally or parenterally in a pharmaceutically effective amount according to a desired method, and the term "pharmaceutically effective amount" of the disclosure means an amount that is sufficient to treat a disease with a reasonable benefit/risk ratio applicable to medical treatment and does not cause side effects, and the effective dose level may be determined according to factors including the patient's health condition, severity, activity of the drug, sensitivity to the drug, administration method, administration time, administration route and excretion rate, treatment period, drugs used in combination or concurrently, and other factors well known in the medical field.

Accordingly, the pharmaceutical composition of the disclosure may be administered to a subject to prevent, treat, and/or diagnose cancer, and the cancer may be skin cancer, breast cancer, uterine cancer, esophageal cancer, gastric cancer, brain tumor, colon cancer, rectal cancer, colorectal cancer, lung cancer, ovarian cancer, cervical cancer, endometrial cancer, vulvar cancer, renal cancer, hematologic cancer, pancreatic cancer, prostate cancer, testicular cancer, laryngeal cancer, head and neck cancer, thyroid cancer, liver cancer, bladder cancer, osteosarcoma, lymphoma, leukemia, thymic cancer, urethral cancer, or bronchial cancer, but is not limited thereto, and preferably, may be a cancer having a characteristic in that acidity is higher than that of normal cells and cytotoxicity is inhibited by a tubulin polymerization inhibitor, and non-limiting examples thereof include breast cancer, preferably HER2-positive breast cancer or triple-negative breast cancer.

In the disclosure, the term "subject" is not limited to a mammal such as livestock or a human that requires prevention, treatment, and/or diagnosis of the cancer, but may preferably be a human.

The pharmaceutical composition of the disclosure may be formulated in various forms for administration to a subject, and a representative form for parenteral administration is an injectable form, preferably an isotonic aqueous solution or suspension. Injectable formulations can be prepared according to techniques known in the art using suitable dispersing or wetting agents and suspending agents. For example, each component can be dissolved in saline or a buffer solution and formulated for injection. In addition, formulations for oral administration include, for example, ingestible tablets, buccal tablets, troches, capsules, elixirs, suspensions, syrups, and wafers, and these formulations may contain, in addition to active ingredients, diluents (e.g., lactose, dextrose, sucrose, mannitol, sorbitol, cellulose and/or glycine) and lubricants (e.g., silica, talc, stearic acid and its magnesium or calcium salts and/or polyethylene glycol). The tablets may contain binding agents such as magnesium aluminum silicate, starch paste, gelatin, tragacanth, methylcellulose, sodium carboxymethylcellulose and/or polyvinylpyrrolidine, and may optionally further contain disintegrants such as starch, agar, alginic acid or its sodium salt, absorbents, colorants, flavoring agents and/or sweetening agents. The formulations may be prepared by conventional mixing, granulation or coating methods.

In addition, the pharmaceutical composition of the disclosure may further include auxiliary agents such as preservatives, wetting agents, emulsifying agents, salts for osmotic pressure control or buffers, and other therapeutically useful substances, and may be formulated according to conventional methods.

The pharmaceutical composition according to the disclosure may be administered through various routes including oral, transdermal, subcutaneous, intravenous or intramuscular routes, and the dosage of the active ingredient may be appropriately selected according to various factors such as the route of administration, the patient's age, sex, weight, and the severity of the patient's condition. In addition, the composition of the disclosure may be administered in combination with known compounds that can enhance the desired effect.

As routes of administration for the pharmaceutical composition according to the disclosure, the pharmaceutical composition may be administered to humans and animals orally or parenterally such as intravenously, subcutaneously, intranasally, or intraperitoneally. Oral administration also includes sublingual application. Parenteral administration includes injection methods such as subcutaneous injection, intramuscular injection, and intravenous injection, and instillation methods.

In the pharmaceutical composition of the disclosure, the total effective amount of the quinazoline-benzimidazole derivative or a pharmaceutically acceptable salt thereof according to the disclosure may be administered to a patient as a single dose, or may be administered by a fractionated treatment protocol in which multiple doses are administered over a long period of time. The pharmaceutical composition of the disclosure may vary the content of the active ingredient depending on the severity of the disease, but may typically be administered several times a day at an effective dose of 100 *µ*g to 3,000 mg per administration for adults. However, regarding the concentration of the quinazoline-benzimidazole derivative or the pharmaceutically acceptable salt thereof, an effective dosage for a patient may be determined by considering various factors such as age, body weight, health condition, sex, severity of the disease, diet, and excretion rate of a patient, as well as a route of administration and a frequency of treatment of the drug.

In addition, the pharmaceutical composition according to the disclosure is not particularly limited in its formulation, administration route, and administration method as long as it exhibits the effects of the disclosure, and the pharmaceutical composition of the disclosure may additionally include a known anticancer agent in addition to the quinazoline-benzimidazole derivative or a pharmaceutically acceptable salt thereof as an active ingredient, and may be used in combination with other known treatments for the treatment of these diseases.

The terminology used herein is for the purpose of describing particular embodiments only and is not to be limiting of the embodiments. The singular forms "a", "an", and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the same meaning as commonly understood by one of ordinary skill in the art to which the examples belong. Terms, such as those defined in commonly used dictionaries, are to be interpreted as having a meaning that is consistent with their meaning in the context of the relevant art and the present disclosure, and are not to be interpreted in an idealized or overly formal sense unless expressly so defined herein.

Hereinafter, embodiments will be described in detail with reference to the accompanying drawings. However, various alterations and modifications may be made to the embodiments. Here, the embodiments are not meant to be limited by the descriptions of the disclosure. The embodiments should be understood to include all changes, equivalents, and replacements within the idea and the technical scope of the disclosure.

When describing the embodiments with reference to the accompanying drawings, like reference numerals refer to like components regardless of drawing numbers and a repeated description related thereto will be omitted. In the description of embodiments, detailed description of well-known related technology will be omitted when it is deemed that such description will cause ambiguous interpretation of the disclosure.

### [EXAMPLES]

### Example 1. Preparation of Quinazoline-Benzimidazole Derivatives

All chemical reagents used were commercially available. ¹H NMR spectra were recorded on a Bruker Avance III 400 MHz, with TMS used as an internal standard.

### 1-1. Synthesis of Methyl (6-(3-(2-morpholinoethyl)-4-oxo-3,4-dihydroquinazolin-6-yl)-1H-benzo[d]imidazol-2-yl)carbamate (Representative Compound Formula 1-1)

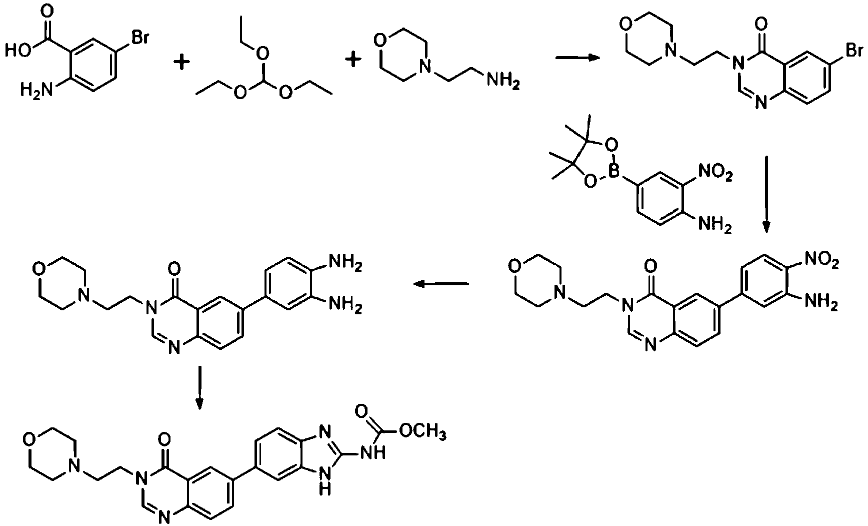

### Step 1] Synthesis of 6-Bromo-3-(2-morpholinoethyl)quinazolin-4(3H)-one

5-Bromoanthranilic acid (2.16 g, 9.3 mmole) was dissolved in 30 ml of anhydrous ethanol under a nitrogen atmosphere. Thereafter, triethyl orthoformate (1.39 g, 13 mmole) and 2-morpholino ethylamine (1.69 g, 13 mmole) were added. Then, iodine (25 mg, 0.1 mmole) was added as a catalyst. Thereafter, the mixture was heated under reflux for 6 hours. When completion of the reaction was confirmed by TLC, the mixture was cooled to room temperature. Then, ethanol was removed by distillation under reduced pressure, and the residue was dissolved in 60 ml of ethyl acetate. Thereafter, the solution was washed with a 1N-NaOH aqueous solution and purified water, respectively, and then dried over anhydrous Na₂SO₄ and filtered. Then, the filtrate was distilled under reduced pressure to obtain a light yellow solid. (2.21 g)
Yield 69.7%,
¹H NMR (400 MHz. CDCl₃): δ 2.51(t, J=3.6 Hz, 4H, 2(N-CH2)), 2.70(t, J=4.76 Hz, 2H, N-CH2), 3.67(t, J=3.6 Hz, 4H, 2(O-CH2)), 4.08(t, J=4.76 Hz, 2H, N-CH2), 7.57~7.84(m, 2H, ArH), 8.05(s, 1H, ArH), 8.43(s, 1H, -NC-H)

### Step 2] Synthesis of 6-(4-Amino-3-nitrophenyl)-3-(2-morpholinoethyl)quinazolin-4(3H)-one (Formula 2-1)

2-Nitro-4-(4,4,5,5-tetramethyl-1,3,2-dioxaborolan-2-yl)aniline (1 g, 3.8 mmole), 6-bromo-3-(2-morpholinoethyl)quinazolin-4(3H)-one (1.28 g, 3.8 mmole), and K2CO3 (1.56 g, 11.4 mmole) were dissolved in 25 ml of dioxane under a nitrogen atmosphere. Thereafter, the mixture was heated to 80°C, and then Pd(dppf)Cl2 (0.14 g, 0.19 mmole) was added, followed by heating under reflux for 6 hours. Completion of the reaction was confirmed by TLC, and the mixture was distilled under reduced pressure. Then, a yellow solid was obtained by purification through column chromatography. (0.58 g)
Yield 38.9%,
¹H NMR (400 MHz. CDCl3): δ 2.55(t, J=3.6 Hz, 4H, 2(N-CH2)), 2.76(t, J=4.76 Hz, 2H, N-CH2), 3.71(t, J=3.6 Hz, 4H, 2(O-CH2)), 4.14(t, J=4.76 Hz, 2H, N-CH2), 6.24(br. s,, 2H, NH2), 6.95~8.49(m, 7H, Aryl-H)

### Step 3] Synthesis of 6-(3,4-Diaminophenyl)-3-(2-morpholinoethyl)quinazolin-4(3H)-one (Formula 3-1)

6-(4-Amino-3-nitrophenyl)-3-(2-morpholinoethyl)quinazolin-4(3H)-one (0.58 g, 1.4 mmole) was dissolved in 20 ml of methanol, and then 0.35 g of 10%-Pd/C was added, followed by stirring at room temperature. Thereafter, hydrazine hydrate (0.75 g, 14 mmole) was added dropwise, followed by heating under reflux for 3 hours. After the reaction was confirmed by TLC, the mixture was cooled to room temperature. Then, the mixture was filtered through Celite, and the filtrate was distilled under reduced pressure. Subsequently, the residue was dissolved again in 20 ml of methylene chloride and dried over anhydrous MgSO4. The resulting mixture was filtered and then distilled under reduced pressure to obtain a yellow solid. (0.39 g)
Yield 73.5%,
¹H NMR (400 MHz. CDCl3): δ 2.55(t, J=3.6 Hz, 4H, 2(N-CH2)), 2.76(t, J=4.76 Hz, 2H, N-CH2), 3.71(t, J=3.6 Hz, 4H, 2(O-CH2)), 4.14(t, J=4.76 Hz, 2H, N-CH2), 6.24(br. s,, 2H, NH2), 6.81~8.47(m, 7H, Aryl-H)

### Step 4] Synthesis of Methyl (6-(3-(2-morpholinoethyl)-4-oxo-3,4-dihydroquinazolin-6-yl)-1H-benzo[d]imidazol-2-yl)carbamate (Formula 1-1)

6-(3,4-Diaminophenyl)-3-(2-morpholinoethyl)quinazolin-4(3H)-one (0.2 g, 0.5 mmole) and 1,3-Bis(methoxycarbonyl)-2-methyl-2-thiopseudourea (0.29 g, 14 mmole) were added to 15 ml of 5%-AcOH in ethanol, followed by heating under reflux for 3 hours. When a precipitate was formed, the mixture was cooled and filtered to obtain a yellow solid. (0.17g)
Yield 73%
¹H NMR (400 MHz. DMSO-d6): δ 2.55(t, J=3.6 Hz, 4H, 2(N-CH2)), 2.76(t, J=4.76 Hz, 2H, N-CH2), 3.71(t, J=3.6 Hz, 4H, 2(O-CH2)), 3.75(s, 3H, -OCH3), 4.14(t, J=4.76 Hz, 2H, N-CH2), 7.50~8.38(m, 7H, Aryl-H)

### 1-2. Synthesis of 3-(2-Morpholinoethyl)-6-(2-(p-tolyl)-1H-benzo[d]imidazol-6-yl)quinazolin-4(3H)-one (Formula 1-2)

6-(3,4-Diaminophenyl)-3-(2-morpholinoethyl)quinazolin-4(3H)-one (0.15 g, 0.4 mmole) and p-methylbenzaldehyde (50 mg, 0.4 mmole) were dissolved in 10 ml of DMF, followed by stirring at 90°C for 5 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid. (0.1 g)
Yield 52.53%
¹H NMR (400 MHz. CDCl3): δ 2.38(s, 3H, -CH3), 2.54(t, J=3.6 Hz, 4H, 2(N-CH2)), 2.76(t, J=4.76 Hz, 2H, N-CH2), 3.69(t, J=3.6 Hz, 4H, 2(O-CH2)), 4.14(t, J=4.76 Hz, 2H, N-CH2), 7.26~8.53(m, 7H, Aryl-H)

### 1-3. Synthesis of 6-(2-(4-Fluorophenyl)-1H-benzo[d]imidazol-6-yl)-3-(2-morpholinoethyl)quinazolin-4(3H)-one (Formula 1-3)

6-(3,4-Diaminophenyl)-3-(2-morpholinoethyl)quinazolin-4(3H)-one (0.15 g, 0.4 mmole) and p-fluorobenzaldehyde (50 mg, 0.4 mmole) were dissolved in 10 ml of DMF, followed by stirring at 90°C for 5 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid.
(0.07 g)
Yield 36.8%,
¹H NMR (400 MHz. DMSO-d6): δ 2.33(t, J=3.6 Hz, 4H, 2(N-CH2)), 2.65(t, J=4.76 Hz, 2H, N-CH2), 3.55(t, J=3.6 Hz, 4H, 2(O-CH2)), 4.13(t, J=4.76 Hz, 2H, N-CH2), 7.41~8.40(m, 11H, Aryl-H), 13.08(br. s,, 1H, NH)

### 1-4. Synthesis of 6-(2-(3-Fluorophenyl)-1H-benzo[d]imidazol-6-yl)-3-(2-morpholinoethyl)quinazolin-4(3H)-one (Formula 1-4)

6-(3,4-Diaminophenyl)-3-(2-morpholinoethyl)quinazolin-4(3H)-one (0.15 g, 0.4 mmole) and m-fluorobenzaldehyde (50 mg, 0.4 mmole) were dissolved in 10 ml of DMF, followed by stirring at 90°C for 5 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid.
(0.048 g)
Yield 22.85%
¹H NMR (400 MHz. DMSO-d6): δ 2.33(t, J=3.6 Hz, 4H, 2(N-CH2)), 2.67(t, J=4.76 Hz, 2H, N-CH2), 3.55(t, J=3.6 Hz, 4H, 2(O-CH2)), 4.13(t, J=4.76 Hz, 2H, N-CH2), 7.35~8.41(m, 11H, Aryl-H), 13.19(br. s,, 1H, NH)

### 1-5. Synthesis of 6-(2-(4-Chlorophenyl)-1H-benzo[d]imidazol-6-yl)-3-(2-morpholinoethyl)quinazolin-4(3H)-one (Formula 1-5)

6-(3,4-Diaminophenyl)-3-(2-morpholinoethyl)quinazolin-4(3H)-one (0.15 g, 0.4 mmole) and p-chlorobenzaldehyde (58 mg, 0.4 mmole) were dissolved in 10 ml of DMF, followed by stirring at 90°C for 5 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid. (0.075 g)
Yield 37.68%,
¹H NMR (400 MHz. DMSO-d6): δ 2.46(t, J=3.6 Hz, 4H, 2(N-CH2)), 2.65(t, J=4.76 Hz, 2H, N-CH2), 3.55(t, J=3.6 Hz, 4H, 2(O-CH2)), 4.14(t, J=4.76 Hz, 2H, N-CH2), 7.62~8.40(m, 11H, Aryl-H), 13.14(br. s, 1H, NH)

### 1-6. Synthesis of 6-(2-(2,4-Dihydroxyphenyl)-1H-benzo[d]imidazol-6-yl)-3-(2-morpholinoethyl)quinazolin-4(3H)-one (Formula 1-6)

6-(3,4-Diaminophenyl)-3-(2-morpholinoethyl)quinazolin-4(3H)-one (0.15 g, 0.4 mmole) and 2,4-dihydroxybenzaldehyde (57 mg, 0.4 mmole) were dissolved in 10 ml of DMF, followed by stirring at 90°C for 5 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid. (0.032 g)
Yield 16%
¹H NMR (400 MHz. DMSO-d6): δ 2.33(t, J=3.6 Hz, 4H, 2(N-CH2)), 2.67(t, J=4.76 Hz, 2H, N-CH2), 3.54(t, J=3.6 Hz, 4H, 2(O-CH2)), 6.41(s, 1H, -OH), 6.46(d, 1H, -OH), 4.13(t, J=4.76 Hz, 2H, N-CH2), 7.62~8.40(m, 11H, Aryl-H), 13.14(br, s, 1H, NH)

### 1-7. Synthesis of 6-(2-(5-Fluoro-2-hydroxyphenyl)-1H-benzo[d]imidazol-6-yl)-3-(2-morpholinoethyl)quinazolin-4(3H)-one (kdnc-2316)

6-(3,4-Diaminophenyl)-3-(2-morpholinoethyl)quinazolin-4(3H)-one (0.15 g, 0.4 mmole) and 3-fluoro-6-hydroxybenzaldehyde (57 mg, 0.4 mmole) were dissolved in 10 ml of DMF, followed by stirring at 90°C for 5 hours. After the reaction was confirmed by TLC, the mixture was cooled, and 30 ml of ethyl acetate was added, followed by washing with 10 ml of purified water three times. Then, the mixture was dried over anhydrous MgSO4, filtered, and concentrated under reduced pressure. Thereafter, the residue was purified by column chromatography to obtain a pale yellow solid. (0.091 g)
Yield 45.73%
¹H NMR (400 MHz. DMSO-d6): δ 2.46(t, J=3.6 Hz, 4H, 2(N-CH2)), 2.63(t, J=4.76 Hz, 2H, N-CH2), 3.54(t, J=3.6 Hz, 4H, 2(O-CH2)), 4.13(t, J=4.76 Hz, 2H, N-CH2), 7.06~8.42(m, 11H, Aryl-H), 13.07(br. s,, 1H, NH)

### 1-8. Synthesis of 6-(2-Cyclohexyl-1H-benzo[d]imidazol-6-yl)-3-(2-morpholinoethyl)quinazolin-4(3H)-one (Formula 1-8)

6-(3,4-Diaminophenyl)-3-(2-morpholinoethyl)quinazolin-4(3H)-one (0.15 g, 0.4 mmole) and Cyclohexylbenzaldehyde (46 mg, 0.4 mmole) were dissolved in 10 ml of DMF, followed by stirring at 90°C for 5 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid. (0.030 g)
Yield 16%,
¹H NMR (400 MHz. DMSO-d6): δ 1.23(m, 2H, J=11 Hz, Hexyl-H), 1.33(m, 2H, J=11 Hz, Hexyl-H), 1.58(m, 2H, J=11 Hz, Hexyl-H), 1.71(m, 2H, J=11 Hz, Hexyl-H), 1.82(m, 2H, J=11 Hz, Hexyl-H) 2.46(t, J=3.6 Hz, 4H, 2(N-CH2)), 2.63(t, J=4.76 Hz, 2H, N-CH2), 2.87(m, 1H, J=11 Hz, Hexyl-H), 3.53(t, J=3.6 Hz, 4H, 2(O-CH2)), 4.12(t, J=4.76 Hz, 2H, N-CH2), 7.49~8.35(m, 7H, Aryl-H), 12.26(br, s, 1H, NH)

### 1-9. Synthesis of 6-(2-Cyclopentyl-1H-benzo[d]imidazol-6-yl)-3-(2-morpholinoethyl)quinazolin-4(3H)-one (Formula 1-9)

6-(3,4-Diaminophenyl)-3-(2-morpholinoethyl)quinazolin-4(3H)-one (0.15 g, 0.4 mmole) and Cyclopentylbenzaldehyde (40 mg, 0.4 mmole) were dissolved in 10 ml of DMF, followed by stirring at 90°C for 5 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid. (0.024 g)
Yield 13.3%
¹H NMR (400 MHz. DMSO-d6): δ 1.65(m, 2H, J=4.4 Hz, pentyl-H), 1.78(m, 2H, J=4.4 Hz, pentyl-H), 1.91(m, 2H, J=4.4 Hz, pentyl-H), 2.09(m, 2H, J=4.4 Hz, pentyl-H), 2.46(t, J=3.6 Hz, 4H, 2(N-CH2)), 2.63(t, J=4.76 Hz, 2H, N-CH2), 3.32(m, 1H, J=4.4 Hz, pentyl-H), 3.53(t, J=3.6 Hz, 4H, 2(O-CH2)), 4.12(t, J=4.76 Hz, 2H, N-CH2), 7.51~8.36(m, 7H, Aryl-H), 12.28(br, s, 1H, NH)

### 1-10. Synthesis of 6-(2-(4-Methoxyphenyl)-1H-benzo[d]imidazoyl3-(2-morpholinoethyl)quinazolin-4(3H)-one (Formula 1-10)

6-(3,4-Diaminophenyl)-3-(2-morpholinoethyl)quinazolin-4(3H)-one (0.13 g, 0.35 mmole) and 2,4-difluorobenzaldehyde (48 mg, 0.35 mmole) were dissolved in 10 ml of DMF, followed by stirring at 90°C for 5 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid. (0.070 g)
Yield 41.17%
¹H NMR (400 MHz. CDCl3): δ 2.46(t, J=3.6 Hz, 4H, 2(N-CH2)), 2.64(t, J=4.76 Hz, 2H, N-CH2), 3.54(t, J=3.6 Hz, 4H, 2(O-CH2)), 3.85(s, 3H, -OCH3) 4.13(t, J=4.76 Hz, 2H, N-CH2), 7.13~8.39(m, 11H, Aryl-H), 12.90(br. s,, 1H, NH),

### 1-11. Synthesis of 6-(2-((3-Chloro-4-fluorophenyl)-1H-benzo[d]imidazoyl3-(2-morpholinoethyl)quinazolin-4(3H)-one (Formula 1-11)

6-(3,4-Diaminophenyl)-3-(2-morpholinoethyl)quinazolin-4(3H)-one (0.15 g, 0.4 mmole) and 3-chloro-4-fluorobenzaldehyde (65 mg, 0.4 mmole) were dissolved in 10 ml of DMF, followed by stirring at 90°C for 5 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid. (0.116 g)
Yield 56.31%
¹H NMR (400 MHz. CDCl3): δ 2.46(t, J=3.6 Hz, 4H, 2(N-CH2)), 2.65(t, J=4.76 Hz, 2H, N-CH2), 3.54(t, J=3.6 Hz, 4H, 2(O-CH2)), 4.13(t, J=4.76 Hz, 2H, N-CH2), 7.63~8.41(m, 10H, Aryl-H), 13.18(br, s, 1H, NH)

### 1-12. Synthesis of 6-(2-((2,4-Difluorophenyl)-1H-benzo[d]imidazoyl3-(2-morpholinoethyl)quinazolin-4(3H)-one (Formula 1-12)

6-(3,4-Diaminophenyl)-3-(2-morpholinoethyl)quinazolin-4(3H)-one (0.15 g, 0.4 mmole) and 2,4-difluorobenzaldehyde (58 mg, 0.4 mmole) were dissolved in 10 ml of DMF, followed by stirring at 90°C for 5 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid. (0.057 g)
Yield 28.5%
¹H NMR (400 MHz. CDCl3): δ 2.45(t, J=3.6 Hz, 4H, 2(N-CH2)), 2.65(t, J=4.76 Hz, 2H, N-CH2), 3.55(t, J=3.6 Hz, 4H, 2(O-CH2)), 4.13(t, J=4.76 Hz, 2H, N-CH2), 7.32~8.40(m, 10H, Aryl-H), 12.8(br s, 1H, NH)

### 1-13. Synthesis of Methyl (6-(4-oxo-3-(2-(piperidin-1-yl)ethyl)-3,4-dihydroquinazolin-6-yl)-1H-benzo[d]imidazol-2-yl)carbamate (Representative Compound Formula 1-13)

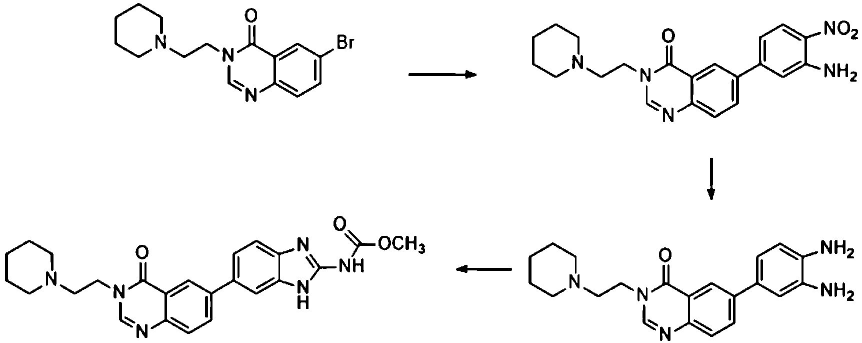

### Step 1] Synthesis of 6-Bromo-3-(2-(piperidin-1-yl)ethyl)quinazolin-4(3H)-one

To 2-amino-5-bromobenzoic acid (2.16 g, 9.4 mmole), 30 ml of anhydrous ethanol was added, and under nitrogen, 1-(2-aminoethyl)piperidine (1.56 g, 12.2 mmole), triethyl orthoformate (1.8 g, 12.2 mmole), and iodine (23 mg, 0.09 mmole) were added in order, followed by heating under reflux for 6 hours. After the reaction was confirmed by TLC, the mixture was cooled to room temperature. Thereafter, all of the ethanol was distilled under reduced pressure, and then 60 ml of ethyl acetate was added, followed by washing with 30 ml of 1N-NaOH three times and with 30 ml of brine three times. The organic layer was dried over anhydrous Na2SO4, filtered, and distilled under reduced pressure to obtain a brown liquid (2.43 g).
Yield 77.14%,
¹H NMR (400 MHz. DMSO-d6): δ 1.32(m, 6H, 3(-CH2)), 2.35(m, 4H, 2(-CH2)), 2.52(t, J=6 Hz, 2H, N-CH2), 4.13(t, J=6 Hz, 2H, N-CH2), 7.57~8.30(m, 4H, Aryl-H)

### Step 2] Synthesis of 6-(4-Amino-3-nitrophenyl)-3-(2-(piperidin-lyl)ethyl)quinazolin-4(3H)-one (Formula 2-2)

6-Bromo-3-(2-(piperidin-1-yl)ethyl)quinazolin-4(3H)-one (1.0 g, 3.78 mmole), 4-amino-3-nitrophenylboronic acid pinacol ester (1.27 g, 3.78 mmole), and potassoim carbonate (1.56 g, 11.3 mmole) were dissolved in 25 ml of dioxane aqueous solution (20 ml of dioxane + 5 ml of purified water), heated to 100°C, and then Pd(dppf)Cl2 (0.14 g, 0.18 mmole) catalyst was added, followed by heating under reflux for 6 hours under a nitrogen atmosphere. After the reaction was confirmed by TLC, the mixture was filtered through Celite to remove solid impurities. An additional 30 ml of ethyl acetate was added, followed by slurrying for 1 hour and filtering to obtain a brown solid.
(0.68 g)
Yield 46%
¹H NMR (400 MHz. DMSO-d6): δ 1.37(m, 6H, 3(-CH2)), 2.39(m, 4H, 2(-CH2)), 2.56(t, J=6 Hz, 2H, N-CH2), 4.09(t, J=6 Hz, 2H, N-CH2), 7.15~8.31(m, 7H, Aryl-H)

### Step 3] Synthesis of 6-(3,4-Diaminophenyl)-3-(2-(piperidin-1-yl)ethyl)quinazolin-4(3H)-one (Formula 3-2)

6-(4-Amino-3-nitrophenyl)-3-(2-(piperidin-lyl)ethyl)quinazolin-4(3H)-one (0.62 g, 1.58 mmole) was dissolved in 20 ml of methanol, and 0.37 g of 10%-Pd/C was added, followed by stirring. Thereafter, hydrazine hydrate (0.79 g, 15.8 mmole) was added, followed by heating under reflux for 4 hours. After the reaction was confirmed by TLC, the mixture was filtered through Celite. The organic layer was dried over anhydrous magnesium sulfate, filtered, and distilled under reduced pressure to obtain a light yellow liquid.
(0.45 g)
Yield 78.9%
¹H NMR (400 MHz. DMSO-d₆): δ 1.37(m, 6H, 3(-CH2)), 2.39(m, 4H, 2(-CH2)), 2.57(t, J=6 Hz, 2H, N-CH2), 4.07(t, J=6 Hz, 2H, N-CH2), 4.65(s, 2H, NH2), 4.74(s, 2H, NH2), 6.59~8.94(m, 7H, Aryl-H)

### Step 4] Synthesis of Methyl 6-(3,4-dihydro-4-oxo-3-(2-piperidin-1-yl)ethyl)quinazoli-6-yl)-1H-benzo[d]imidazol -2-yl carbamate (Formula 1-13)

6-(3,4-Diaminophenyl)-3-(2-(piperidin-1-yl)ethyl)quinazolin-4(3H)-one (0.2 g, 0.5 mmole) and 1,3-Bis(methoxycarbonyl)-2-methyl-2-thiopseudourea (0.29 g, 14 mmole) were added to 15 ml of 5%-AcOH in ethanol, followed by heating under reflux for 3 hours. When a precipitate was formed, the mixture was cooled and filtered to obtain a yellow solid. (0.177 g)
Yield 72%
¹H NMR (400 MHz. CDCl3): δ 1.37(br. s,, 2H, cycloalkyl-CH2) 1.45(br. s,, 4H, cycloalkyl-CH2) 2.40(br. s,, 4H, cycloalkyl N-CH2), 2.67(t, J=4.76 Hz, 2H, N-CH2), 3.80(s, 3H, OCH3), 4.10(t, J=4.76 Hz, 2H, N-CH2), 7.49~8.33(m, 7H, Aryl-H), 11.68(br. s,, 1H, NH),

### 1-14. Synthesis of 6-(2-(4-Fluorophenyl)-1H-benzo[d]imidazol-6-yl)-3-(2-(piperidin-1-yl)ethyl)quinazolin-4(3H)-one (Formula 1-14)

6-(3,4-Diaminophenyl)-3-(2-(piperidin-1-yl)ethyl)quinazolin-4(3H)-one (0.2 g, 0.55 mmole) and 4-fluorobenzaldehyde (68 mg, 0.55 mmole) were dissolved in 10 ml of DMF, followed by stirring at 90°C for 5 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid. (0.080 g)
Yield 42.1%
¹H NMR (400 MHz. CDCl3): δ 1.37(br. s, 2H, cycloalkyl-CH2) 1.46(br. s,, 4H, cycloalkyl-CH2) 2.41(br, s. 4H, cycloalkyl N-CH2), 2.68(t, J=4.76 Hz, 2H, N-CH2), 4.11(t, J=4.76 Hz, 2H, N-CH2), 7.42~8.40(m, 11H, Aryl-H), 13.10(br. s, 1H, NH),

### 1-15. Synthesis of 6-(2-(4-Chlorophenyl)-1H-benzo[d]imidazol-6-yl)-3-(2-(piperidin-1-yl)ethyl)quinazolin-4(3H)-one (Formula 1-15)

6-(3,4-Diaminophenyl)-3-(2-(piperidin-1-yl)ethyl)quinazolin-4(3H)-one (0.2 g, 0.55 mmole) and 4-chlorobenzaldehyde (77 mg, 0.55 mmole) were dissolved in 10 ml of DMF, followed by stirring at 90°C for 5 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid. (0.103 g)
Yield 38.72%
¹H NMR (400 MHz. CDCl3): δ 1.37(br. s,, 2H, cycloalkyl-CH2) 1.45(br. s,, 4H, cycloalkyl-CH2) 2.41(br. s,, 4H, cycloalkyl N-CH2), 2.58(t, J=4.76 Hz, 2H, N-CH2), 4.11(t, J=4.76 Hz, 2H, N-CH2), 7.62~8.40(m, 11H, Aryl-H), 13.23(br. s,, 1H, NH)

### 1-16. Synthesis of 6-(2-(3-Chloro-4-chlorophenyl)-1H-benzo[d]imidazol-6-yl)-3-(2-(piperidin-1-yl)ethyl)quinazolin-4(3H)-one (Formula 1-16)

6-(3,4-Diaminophenyl)-3-(2-(piperidin-1-yl)ethyl)quinazolin-4(3H)-one (0.2 g, 0.55 mmole) and 3-chloro-4-fluorobenzaldehyde (87 mg, 0.55 mmole) were dissolved in 10 ml of DMF, and then sodium metabisulfite (Na₂S₂O₅, 114 mg) was added, followed by stirring at 95°C for 3 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by silica gel column chromatography sequentially eluting with a mixed developing solution of methylene chloride and methanol (5:1, v/v), methylene chloride and methanol (3:1, v/v), and ethyl acetate and methanol (5:1, v/v), respectively, to obtain a pale yellow solid. (0.048 g)
Yield 17.39%
¹H NMR (400 MHz. CDCl3) : δ 1.38(br. s,, 2H, cycloalkyl-CH2) 1.46(br. s,, 4H, cycloalkyl-CH2) 2.41(br. s,, 4H, cycloalkyl N-CH2), 2.68(t, J=4.76 Hz, 2H, N-CH2), 4.11(t, J=4.76 Hz, 2H, N-CH2), 7.64~8.40(m, 10H, Aryl-H), 13.19(br. s,, 2H, NH)

### 1-17. Synthesis of 6-(2-(2,4-Difliorophenyl)-1H-benzo[d]imidazol-6-yl)-3-(2-(piperidin-1-yl)ethyl)quinazolin-4(3H)-one (Formula 1-17)

6-(3,4-Diaminophenyl)-3-(2-(piperidin-1-yl)ethyl)quinazolin-4(3H)-one (0.2 g, 0.55 mmole) and 2,4-fluorobenzaldehyde (78 mg, 0.55 mmole) were dissolved in 10 ml of DMF, followed by stirring at 90°C for 5 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid. (0.083 g)
Yield 31.08%
¹H NMR (400 MHz. CDCl3): δ 1.36(br. s,, 2H, cycloalkyl-CH2) 1.45(br. s,, 4H, cycloalkyl-CH2) 2.40(br. s,, 4H, cycloalkyl N-CH2), 2.58(t, J=4.76 Hz, 2H, N-CH2), 4.11(t, J=4.76 Hz, 2H, N-CH2), 7.31~8.40(m, 10H, Aryl-H), 12.74(br. s,, 2H, NH)

### 1-18. Synthesis of 6-(2-(4-Methoxyphenyl)-1H-benzo[d]imidazol-6-yl)-3-(2-(piperidin-1-yl)ethyl)quinazolin-4(3H)-one (Formula 1-18)

6-(3,4-Diaminophenyl)-3-(2-(piperidin-1-yl)ethyl)quinazolin-4(3H)-one (0.2 g, 0.55 mmole) and 4-methoxybenzaldehyde (75 mg, 0.55 mmole) were dissolved in 10 ml of DMF, followed by stirring at 90°C for 5 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid. (0.084 g)
Yield 31.93%
¹H NMR (400 MHz. CDCl3): δ 1.36 (br. s,, 2H, cycloalkyl-CH2) 1.45 (br. s,, 4H, cycloalkyl-CH2) 2.40(br. s,, 4H, cycloalkyl N-CH2), 2.58(t, J=4.76 Hz, 2H, N-CH2), 3.85(s, 3H, OCH3), 4.12(t, J=4.76 Hz, 2H, N-CH2), 7.12~8.39(m, 11H, Aryl-H), 12.91(br. s,, 1H, NH)

### 1-19. Synthesis of 3-(2-Piperidin-1-yl)ethyl)-6-(2-p-toyl-1H-benzo[d]imidazol-6-yl)quinazolin-4(3H)-one (Formula 1-19)

6-(3,4-Diaminophenyl)-3-(2-(piperidin-1-yl)ethyl)quinazolin-4(3H)-one (0.15 g, 0.41 mmole) and 4-methylbenzaldehyde (49 mg, 0.41 mmole) were dissolved in 10 ml of DMF, followed by stirring at 90°C for 5 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid. (0.080 g)
Yield 42.1%
¹H NMR (400 MHz. CDCl3): δ 1.38 (br. s,, 2H, cycloalkyl-CH2) 1.45 (br. s,, 4H, cycloalkyl-CH2) 2.40(br. s,, 4H, cycloalkyl N-CH2), 2.41(s, 3H, -CH3), 4.10(t, J=4.76 Hz, 2H, N-CH2), 7.38~8.39(m, 11H, Aryl-H), 12.98(br. s,, 1H, NH)

### 1-20. Synthesis of 3-(2-(Piperidin-1-yl)ethyl)-6-(2-(thiophen-3-yl)-1H-benzo[d]imidazol-6-yl)quinazolin-4(3H)-one (Formula 1-20)

6-(3,4-Diaminophenyl)-3-(2-(piperidin-1-yl)ethyl)quinazolin-4(3H)-one (0.20 g, 0.55 mmole) and 3-Thiophenecarboxaldehyde (62 mg, 0.55 mmole) were dissolved in 10 ml of DMF, followed by stirring at 90°C for 5 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid. (0.10 g)
Yield 40.0%
¹H NMR (400 MHz. DMSO-d6): δ 1.38 (m, 6H, 3(-CH2)), 2.33(m, 4H, 2(-CH2)), 2.67(t, J=6 Hz, 2H, N-CH2), 4.13(t, J=6 Hz, 2H, N-CH2), 7.57~8.40(m, 10H, Aryl-H), 12.95(br. s,, 1H, NH)

### 1-21. Synthesis of 3-(2-(Piperidin-1-yl)ethyl)-6-(2-(pyridin-4-yl)-1H-benzo[d]imidazol-6-yl)quinazolin-4(3H)-one (Formula 1-21)

6-(3,4-Diaminophenyl)-3-(2-(piperidin-1-yl)ethyl)quinazolin-4(3H)-one (0.2 g, 0.55 mmole) and 4-pyridinecarboxaldehyde (59 mg, 0.55 mmole) were dissolved in 10 ml of DMF, followed by stirring at 90°C for 5 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid. (0.03 g)
Yield 12.14%
¹H NMR (400 MHz. DMSO-d6): δ 1.38 (m, 6H, 3(-CH2)), 2.33(m, 4H, 2(-CH2)), 2.67(t, J=6 Hz, 2H, N-CH2), 4.13(t, J=6 Hz, 2H, N-CH2), 7.67~8.80(m, 11H, Aryl-H), 13.43(br. s,, 1H, NH)

### 1-22. Synthesis of 6-(2-(5-Chloropyridin-2-yl)-1H-benzo[d]imidazol-6-yl)-3-(2-(piperidin-1-yl)ethyl)quinazolin-4(3H)-one (Formula 1-22)

6-(3,4-Diaminophenyl)-3-(2-(piperidin-1-yl)ethyl)quinazolin-4(3H)-one (0.2 g, 0.55 mmole) and 5-chloro-2-pyridinecarboxaldehyde (78 mg, 0.55 mmole) were dissolved in 10 ml of DMF, followed by stirring at 90°C for 5 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid.
(0.148 g)
Yield 55.63%
¹H NMR (400MHz. DMSO-d6): δ 1.38(m, 6H, 3(-CH2)), 2.33(m, 4H, 2(-CH2)), 2.60(t, J=6 Hz, 2H, N-CH2), 4.11(t, J=6 Hz, 2H, N-CH2), 7.66~8.83(m, 10H, Aryl-H), 13.35(br. s,, 1H, NH)

### 1-23. Synthesis of 3-(2-Morpholinoethyl)-6-(2-(thiophen-3-yl)-1H-benzo[d]imidazol-6-yl)quinazolin-4(3H)-one (Formula 1-23)

6-(3,4-Diaminophenyl)-3-(2-morpholinoethyl)quinazolin-4(3H)-one (0.20 g, 0.55 mmole) and 3-thiophenecarboxaldehyde (61 mg, 0.55 mmole) were dissolved in 10 ml of DMF, followed by stirring at 90°C for 5 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid. (0.15 g)
Yield 60.0%
¹H NMR (400 MHz. DMSO-d6): δ 2.46(t, J=3.6 Hz, 4H, 2(N-CH2)), 2.65(t, J=4.46 Hz, 2H, N-CH2), 3.54(t, J=4.4 Hz, 4H, 2(O-CH2)), 4.13(t, J=6 Hz, 2H, N-CH2), 7.57~8.39(m, 10H, Aryl-H), 12.95(br. s,, 1H, NH)

### 1-24. Synthesis of 3-(2-Morpholinoethyl)-6-(2-(pyridin-4-yl)-1H-benzo[d]imidazol-6-yl)quinazolin-4(3H)-one (Formula 1-24)

6-(3,4-Diaminophenyl)-3-(2-morpholinoethyl)quinazolin-4(3H)-one (0.2 g, 0.55 mmole) and 4-pyridinecarboxaldehyde (58 mg, 0.55 mmole) were dissolved in 10 ml of DMF, followed by stirring at 90°C for 5 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid. (0.02 g)
Yield 8.1%
¹H NMR (400 MHz. DMSO-d6): δ 2.46(t, J=3.6 Hz, 4H, 2(N-CH2)), 2.65(t, J=4.46 Hz, 2H, N-CH2), 3.54(t, J=4.4 Hz, 4H, 2(O-CH2)), 4.13(t, J=6 Hz, 2H, N-CH2), 7.67~8.8(m, 11H, Aryl-H), 13.43(br. s,, 1H, NH)

### 1-25. Synthesis of 6-(2-(5-Chloropyridin-2-yl)-1H-benzo[d]imidazol-6-yl)-3-(2-morpholinoethyl)quinazolin-4(3H)-one (Formula 1-25)

6-(3,4-Diaminophenyl)-3-(2-morpholinoethyl)quinazolin-4(3H)-one (0.22 g, 0.55 mmole) and 5-chloro-2-pyridinecarboxaldehyde (77 mg, 0.55 mmole) were dissolved in 10 ml of DMF, followed by stirring at 90°C for 5 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid.
(0.052 g)
Yield 19.54%
¹H NMR (400 MHz. DMSO-d6): δ 2.46(t, J=3.6 Hz, 4H, 2(N-CH2)), 2.65(t, J=4.46 Hz, 2H, N-CH2), 3.54(t, J=4.4 Hz, 4H, 2(O-CH2)), 4.13(t, J=6 Hz, 2H, N-CH2), 7.63~8.83(m, 10H, Aryl-H), 13.35(br. s,, 1H, NH)

### 1-26. Synthesis of 6-(2-(3-chloro-4-fluorophenyl)-1H-benzo[d]imidazol-6-yl)-3-(2-(4-methylpiperidin-1-yl)ethyl)quinazolin-4(3H)-one (Representative Compound Formula 1-26)

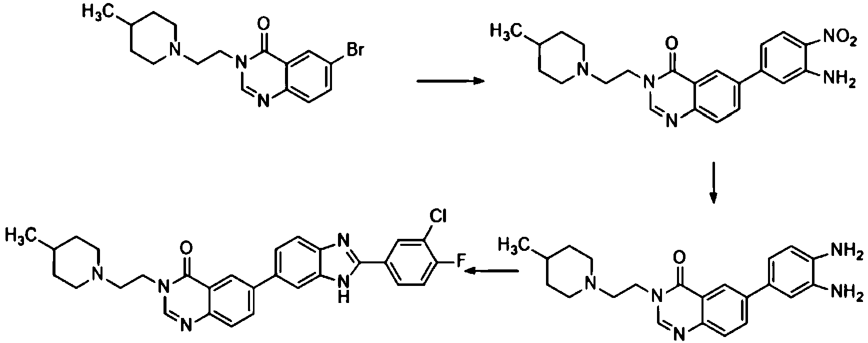

### Step 1] Synthesis of 6-Bromo-3-(2-(4-methylpiperidin-1-yl)ethyl)quinazolin-4(3H)-one

2-Amino-5-bromobenzoic acid (1.26 g, 5.5 mmole) and 2-(4-methyl-1-piperidinyl)ethanamine (1.0 g, 7.0 mmole) were added to 30 ml of anhydrous ethanol under a nitrogen atmosphere. Triethyl orthoformate (1.05 g, 7.0 mmole) and iodine (14 mg, 0.01 eq) were added, followed by heating under reflux for 6 hours. After the reaction was confirmed by TLC, the mixture was slowly cooled. Then, the solvent was completely removed by distillation under reduced pressure. Thereafter, the residue was diluted in 60 ml of ethyl acetate and washed with a IN-NaOH solution and brine. Anhydrous Na₂SO₄ was added for drying, followed by vacuum filtration. The filtrate was distilled under reduced pressure to completely remove the solvent, and a brown liquid was obtained.
(1.69 g)
Yield 88.48%
¹H NMR (400 MHz. DMSO-d6): δ 0.85(d, J=6.4 Hz, 3H, -CH3), 1.01(m, 2H, -CH2), 1.27(m, 1H, -CH), 1.54(m, 2H, -CH2), 1.93(m, 2H, -CH2), 2.55(t, J=6 Hz, 2H, -CH2), 2.83(m, 2H, -CH2), 4.06(t, J=6 Hz, 2H, -CH2), 7.61~8.32(m, 4H, Aryl-H)

### Step 2] Synthesis of 6-(4-Amino-3-nitrophenyl)-3-(2-(4-methylpiperidin-1-yl)ethyl)quinazolin-4(3H)-one (Formula 2-3)

2-Nitro-4-(4,4,5,5-tetramethyl-1,3,2-dioxaborolan-2-yl)aniline (1.27 g, 4.8 mmole), 6-bromo-3-(2-(4-methylpiperidin-1-yl)ethyl)quinazolin-4(3H)-one (1.68 g, 4.8 mmole), and K2CO3 (1.99 g, 14.4 mmole) were added, and 25 ml of wet dioxane was added, followed by stirring under a nitrogen atmosphere.

Thereafter, [1,1'-bis(diphenylphosphino)ferrocene]palladium(II) dichloride (0.175 g, 0.05 eq) was added, followed by heating under reflux for 5 hours. Then, the reaction was confirmed by TLC, and the mixture was slowly cooled. After cooling, the mixture was filtered through Celite, and the filtrate was distilled under reduced pressure. Subsequently, ethyl acetate was added, and the mixture was stirred and then filtered to obtain a brown solid.
(0.71 g)
Yield 36.4%
¹H NMR (400 MHz. DMSO-d6): δ 0.85(d, J=6.4 Hz, 3H, -CH3), 1.07(m, 2H, -CH2), 1.30(m, 1H, -CH), 1.56(m, 2H, -CH2), 1.96(m, 2H, -CH2), 2.58(t, J=6 Hz, 2H, -CH2), 2.87(m, 2H, -CH2), 4.09(t, J=6 Hz, 2H, -CH2), 7.16~8.31(m, 7H, Aryl-H)

### Step 3] Synthesis of 6-(3,4-Diaminophenyl)-3-(2-(4-methylpiperidin-1-yl)ethyl)quinazolin-4(3H)-one (Formula 3-3)

6-(4-Amino-3-nitrophenyl)-3-(2-(4-methylpiperidin-1-yl)ethyl)quinazolin-4(3H)-one (0.71 g, 1.74 mmole) was dissolved in 28 ml of methanol, and 0.35 g of 10%-Pd/C was added, followed by stirring. Thereafter, hydrazine hydrate (0.84 g, 17.4 mmole) was added, followed by heating under reflux for 7 hours. After the reaction was confirmed by TLC, the mixture was cooled to room temperature. The mixture was filtered through Celite and then distilled under reduced pressure. The residue was dissolved in 20 ml of methylene chloride and dried using anhydrous magnesium sulfate. The resulting mixture was filtered and distilled under reduced pressure to obtain a brown liquid.
(0.27 g)
Yield 41.5%
¹H NMR (400 MHz. DMSO-d6): δ 0.85(d, 3H, -CH3), 1.05(m, 2H, -CH2), 1.29(m, 1H, CH-CH3), 1.55(m, 2H, -CH2), 1.95(m, 2H, -CH2), 2.58(t, J=6 Hz, 2H, -CH2), 2.86(m, 2H, -CH2), 4.07(t, J=6 Hz, 2H, -CH2), 4.64(s, 2H, -NH2), 4.73(s, 2H, -NH2), 6.60~8.21(m, 7H, Aryl-H)

### Step 4] Synthesis of 6-(2-(3-Chloro-4-fluorophenyl)-1H-benzo[d]imidazol-6-yl)-3-(2-(4-methylpiperidin-1-yl)ethyl)quinazolin-4(3H)-one (Formula 1-26)

6-(3,4-Diaminophenyl)-3-(2-(4-methylpiperidin-1-yl)ethyl)quinazolin-4(3H)-one (0.135 g, 0.35 mmole) and 3-chloro-4-fluorobenzaldehyde (56 mg, 0.35 mmole) were dissolved in 10 ml of DMF, and then sodium metabisulfite (Na₂S₂O₅, 85 mg) was added, followed by stirring at 95°C for 3 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid.
(55 mg)
Yield 30.5%
¹H NMR (400 MHz. DMSO-d6): δ 0.87(d, 3H, -CH3), 1.05(m, 2H, CH2), 1.40(m, 1H, -CH-CH3), 1.99(m, 2H, -CH2), 2.33(m, 4H, 2(-CH2)), 2.67(t, J=6 Hz, 2H, N-CH2), 2.86(m, 2H, CH2), 4.11(t, J=6 Hz, 2H, N-CH2), 7.64~8.41(m, 10H, Aryl-H), 13.19(br. s,, 1H, NH)

### 1-27. Synthesis of 6-(2-(4-Chlorophenyl)-1H-benzo[d]imidazol-6-yl)-3-(2-(4-methylpiperidin-1-yl)ethyl)quinazolin-4(3H)-one(Formula 1-27)

6-(3,4-Diaminophenyl)-3-(2-(4-methylpiperidin-1-yl)ethyl)quinazolin-4(3H)-one (0.135 g, 0.35 mmole) and 4-chlorobenzaldehyde (50 mg, 0.35 mmole) were dissolved in 10 ml of DMF, and then sodium metabisulfite (Na₂S₂O₅, 85 mg) was added, followed by stirring at 95°C for 3 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid. (64 mg)
Yield 35.9%
¹H NMR (400 MHz. DMSO-d6): δ 0.87(d, 3H, -CH3), 1.10(m, 2H, CH2), 1.35(m, 1H, -CH-CH3), 1.56(m, 2H, CH2), 1.99(m, 2H, -CH2), 2.33(m, 4H, 2(-CH2)), 2.67(t, J=6 Hz, 2H, N-CH2), 2.86(m, 2H, CH2), 4.10(t, J=6 Hz, 2H, N-CH2), 7.64~8.41(m, 11H, Aryl-H), 13.16(br. s,, 1H, NH)

### 1-28. Synthesis of 6-(2-(3-chloro-4-fluorophenyl)-1H-benzo[d]imidazol-6-yl)-3-(2-(4-hydroxypiperidin-1-yl)ethyl)quinazolin-4(3H)-one (Representative Compound Formula 1-28)

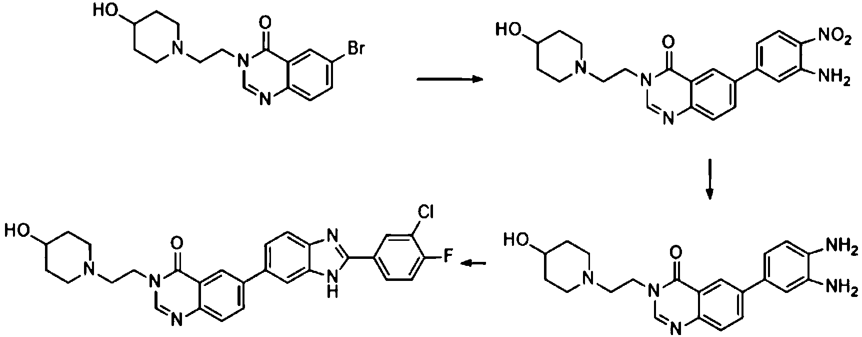

### Step 1] Synthesis of 6-Bromo-3-(2-(4-hydroxypiperidin-1-yl)ethyl)quinazolin-4(3H)-one

2-Amino-5-bromobenzoic acid (1.26 g, 5.5 mmole) and 1-(2-aminoethyl)-4-piperidinol (1.0 g, 7.0 mmole) were added to 30 ml of anhydrous ethanol under a nitrogen atmosphere. Triethyl orthoformate (1.05 g, 7.0 mmole) and iodine (14 mg, 0.01 eq) were added, followed by heating under reflux for 6 hours. After the reaction was confirmed by TLC, the mixture was slowly cooled. Then, the solvent was completely removed by distillation under reduced pressure. Thereafter, the residue was diluted in 60 ml of ethyl acetate and washed with a 1N-NaOH solution and brine. Anhydrous Na₂SO₄ was added for drying, followed by vacuum filtration. The filtrate was distilled under reduced pressure to completely remove the solvent, and a pale yellow solid was obtained.
(1.33 g)
Yield 69.2%
¹H NMR (400 MHz. DMSO-d6): δ 1.28(m, 2H, -CH2), 1.69(m, 2H, -CH2), 2.08(m, 2H, -CH2), 2.55(t, J=6 Hz, 2H, -CH2), 2.71(m, 2H, -CH2), 3.42(m, 1H, CH-OH), 4.06(t, J=6 Hz, 2H, -CH2), 4.52(d, J=4.4 Hz, 1H, -OH), 7.63~8.33(m, 4H, Aryl-H)

### Step 2] Synthesis of 6-(4-Amino-3-nitrophenyl)-3-(2-(4-hydroxypiperidin-1-yl)ethyl)quinazolin-4(3H)-one (Formula 2-4)

6-Bromo-3-(2-(4-hydroxypiperidin-1-yl)ethyl)quinazolin-4(3H)-one (1.33 g, 3.78 mmole), 2-nitro-4-(4,4,5,5-tetramethyl-1,3,2-dioxaborolan-2-yl)aniline (1.0 g, 3.78 mmole), K2CO3 (1.56 g, 11.3 mmole), and Pd(dppf)Cl2 (0.13 g, 0.18 mmole) were added to 25 ml of wet dioxane, followed by stirring under a nitrogen atmosphere.

Thereafter, [1,1'-bis(diphenylphosphino)ferrocene]palladium(II) dichloride (0.13 g, 0.05 eq) was added, followed by heating under reflux for 5 hours. Then, the reaction was confirmed by TLC, and the mixture was slowly cooled. After cooling, the mixture was filtered through Celite, and the filtrate was distilled under reduced pressure. Subsequently, ethyl acetate was added, and the mixture was stirred and then filtered to obtain a reddish-brown solid.
(1.2 g)
Yield 77.4%
¹H NMR (400 MHz. DMSO-d6): δ 1.31(m, 2H, (-CH2)), 1.65(m, 2H, (-CH2)), 2.09(m, 2H, (-CH2)), 2.58(t, J=6 Hz, 2H, N-CH2), 2.74(m, 2H, (-CH2)), 4.08(t, J=6 Hz, 2H, N-CH2), 4.53(br. s,, 1H, -OH), 7.15~8.31(m, 7H, Aryl-H)

### Step 3] Synthesis of 6-(3,4-Diaminophenyl)-3-(2-(4-hydroxypiperidin-1-yl)ethyl)quinazolin-4(3H)-one (Formula 3-4)

6-(4-Amino-3-nitrophenyl)-3-(2-(4-hydroxypiperidin-1-yl)ethyl)quinazolin-4(3H)-one (1.55 g, 3.7 mmole) was dissolved in 80 ml of methanol, and 0.77 g of 10%-Pd/C was added, followed by stirring. Thereafter, hydrazine hydrate (1.89 g, 37.8 mmole) was added, followed by heating under reflux for 7 hours. After the reaction was confirmed by TLC, the mixture was cooled to room temperature. The mixture was filtered through Celite and then distilled under reduced pressure. The residue was dissolved in 20 ml of methylene chloride and dried using anhydrous magnesium sulfate. The resulting mixture was filtered and distilled under reduced pressure to obtain a brown solid.
(1.16 g)
Yield 80.5%
¹H NMR (400 MHz. DMSO-d6): δ 1.32(m, 2H, (-CH2)), 1.69(m, 2H, (-CH2)), 2.13(m, 2H, (-CH2)), 2.61(t, J=6 Hz, 2H, N-CH2), 2.78(m, 2H, (-CH2)), 4.08(t, J=6 Hz, 2H, N-CH2), 4.59(m, 4H, 2(NH2)), 4.72(br. s,, 1H, -OH), 6.60~8.23(m, 7H, Aryl-H)

### Step 4] Synthesis of 6-(2-(3-Chloro-4-fluorophenyl)-1H-benzo[d]imidazol-6-yl)-3-(2-(4-hydroxypiperidin-1-yl)ethyl)quinazolin-4(3H)-one(Formula 1-28)

6-(3,4-Diaminophenyl)-3-(2-(4-hydroxypiperidin-1-yl)ethyl)quinazolin-4(3H)-one (0.15 g, 0.39 mmole) and 3-chloro-4-fluorobenzaldehyde (63 mg, 0.39 mmole) were dissolved in 10 ml of DMF, and then sodium metabisulfite (Na₂S₂O₅, 100 mg) was added, followed by stirring at 95°C for 3 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a yellow solid.
(33 mg)
Yield 16.1%
¹H NMR (400 MHz. DMSO-d6): δ 1.31(m, 2H, (-CH2)), 1.69(m, 2H, (-CH2)), 2.11(m, 2H, (-CH2)), 2.61(t, J=6 Hz, 2H, N-CH2), 2.77(m, 2H, (-CH2)), 3.41(m, 1H, -CH-OH), 4.10(t, J=6 Hz, 2H, N-CH2), 4.55(d, 1H, -OH), 7.64~8.42(m, 10H, Aryl-H), 13.20(br. s,, 1H, NH)

### 1-29. Synthesis of 6-(2-(4-Chlorophenyl)-1H-benzo[d]imidazol-6-yl)-3-(2-(4-hydroxypiperidin-1-yl)ethyl)quinazolin-4(3H)-one (Formula 1-29)

6-(3,4-Diaminophenyl)-3-(2-(4-hydroxypiperidin-1-yl)ethyl)quinazolin-4(3H)-one (0.15 g, 0.39 mmole) and 4-chlorobenzaldehyde (56 mg, 0.39 mmole) were dissolved in 10 ml of DMF, and then sodium metabisulfite (Na₂S₂O₅, 100 mg) was added, followed by stirring at 95°C for 3 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid. (0.48 mg)
Yield 24.3%
¹H NMR (400 MHz. DMSO-d6): δ 1.31(m, 2H, (-CH2)), 1.69(m, 2H, (-CH2)), 2.10(m, 2H, (-CH2)), 2.60(t, J=6 Hz, 2H, N-CH2), 2.77(m, 2H, (-CH2)), 4.09(t, J=6 Hz, 2H, N-CH2), 4.57(s, 1H, -OH), 7.61~8.39(m, 11H, Aryl-H), 13.24(br. s,, 1H, NH)

### 1-30. Synthesis of 3-(2-(4-Hydroxypiperidin-1-yl)ethyl)-6-(2-p-tolyl-1H-benzo[d]imidazol-6-yl)quinazolin-4(3H)-one (Formula 1-30)

6-(3,4-Diaminophenyl)-3-(2-(4-hydroxypiperidin-1-yl)ethyl)quinazolin-4(3H)-one (0.15 g, 0.39 mmole) and 4-methylbenzaldehyde (47 mg, 0.39 mmole) were dissolved in 10 ml of DMF, and then sodium metabisulfite (Na₂S₂O₅, 100 mg) was added, followed by stirring at 95°C for 3 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid. (20 mg)
Yield 10.5%
¹H NMR (400 MHz. DMSO-d6): δ 1.31(m, 2H, (-CH2)), 1.69(m, 2H, (-CH2)), 2.10(m, 2H, (-CH2)), 2.40(s, 3H, -CH3), 2.59(t, J=6 Hz, 2H, N-CH2), 2.76(m, 2H, (-CH2)), 4.10(t, J=6 Hz, 2H, N-CH2), 4.55(s, 1H, -OH), 7.37~8.39(m, 11H, Aryl-H), 13.02(br. s,, 1H, NH)

### 1-31. Synthesis of 3-(2-(4-Hydroxypiperidin-1-yl)ethyl)-6-(2-(4-methoxyphenyl)-1H-benzo[d]imidazol-6-yl)quinazolin-4(3H)-one (Formula 1-31)

6-(3,4-Diaminophenyl)-3-(2-(4-hydroxypiperidin-1-yl)ethyl)quinazolin-4(3H)-one (0.15 g, 0.39 mmole) and 4-methoxybenzaldehyde (54 mg, 0.39 mmole) were dissolved in 10 ml of DMF, followed by stirring at 90°C for 5 hours. After the reaction was confirmed by TLC, the mixture was cooled, and 30 ml of ethyl acetate was added, followed by washing with 10 ml of purified water three times. Then, the mixture was dried over anhydrous MgSO₄, filtered, and concentrated under reduced pressure. Thereafter, the residue was purified by column chromatography to obtain a pale yellow solid.
(21 mg)
Yield 10.7%
¹H NMR (400 MHz. DMSO-d6): δ 1.34(m, 2H, (-CH2)), 1.69(m, 2H, (-CH2)), 2.10(m, 2H, (-CH2)), 2.59(t, J=6 Hz, 2H, N-CH2), 2.76(m, 2H, (-CH2)), 3.85(s, 3H, -OCH3), 4.10(t, J=6 Hz, 2H, N-CH2), 4.56(s, 1H, -OH), 7.12~8.39(m, 11H, Aryl-H), 12.96(br. s,, 1H, NH)

### Example 2. Measurement of Anticancer Activity and Confirmation of Mechanism of Action of Quinazoline-Benzimidazole Derivatives

### 2.1 Screening of Cell Viability of Quinazoline-Benzimidazole Derivatives

MDA-MB-231 (Cell seeding numbers: 0.8(M231) x 10⁴ cells/wells (confluency ≧ 25%), which is a human triple-negative breast cancer (TNBC) cell line, and JIMT-1 (Cell seeding numbers: 0.8(JIMT) x 10⁴ cells/wells (confluency ≧ 25%)), which is a HER2 positive breast cancer (HER2+ BC) cell line, were used in the experiment.

The above cell lines were cultured in Dulbecco's modified Eagle's medium (DMEM) containing 10% fetal bovine serum (FBS), streptomycin-penicillin (100 U/mL), and Fungizone (0.625 *µ*g/mL) at 5% CO₂ and 37°C.

The human breast cancer cell lines MDA-MB-231 and JIMT-1 were treated with quinazoline-benzimidazole derivatives of Formulas 1-1 to 1-31 at various concentrations of 0, 1, and 5 µM, respectively, for 72 hours, and then cell viability was measured using the MTS assay technique. In the MTS assay, cells were attached to a 96-well plate for 24 hours, treated with the quinazoline-benzimidazole derivative for 72 hours, developed with MTS (3-(4,5-dimethylthiazol-2-yl)-5-(3-carboxymethoxyphenyl)-2-(4-sulfophenyl)-2H-tetrazolium) for 4 hours, and then absorbance was measured at 490 nm using a Spectramax Plus384 microplate analyzer.

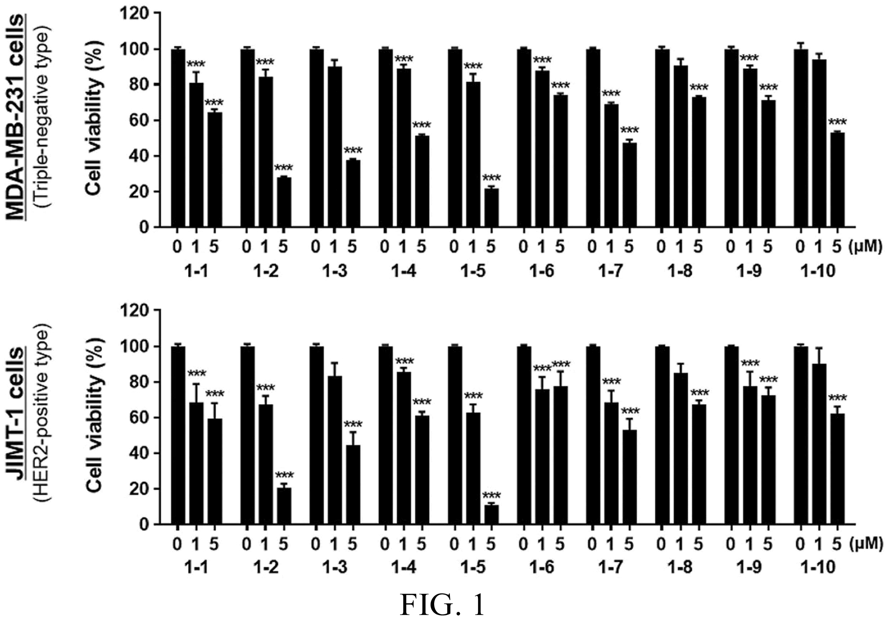

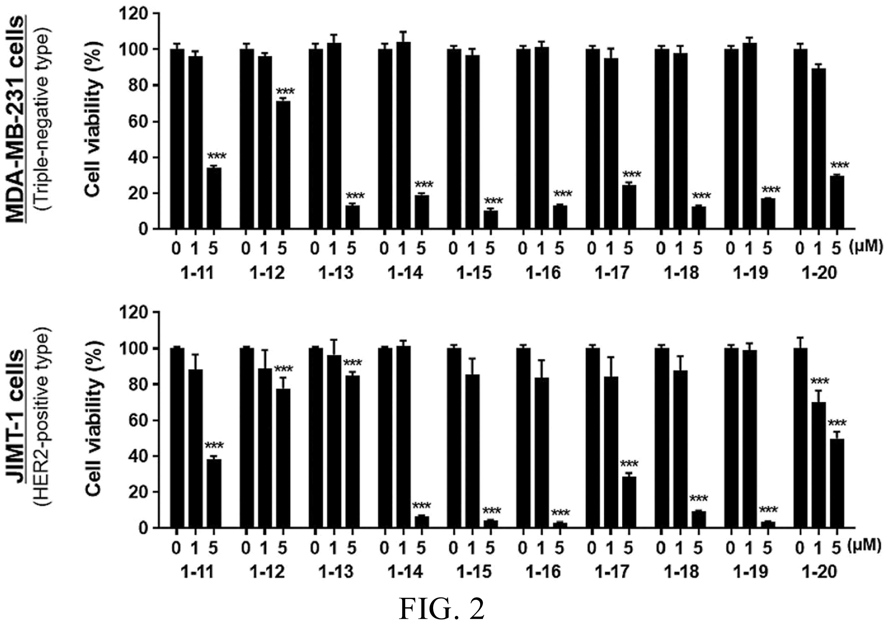

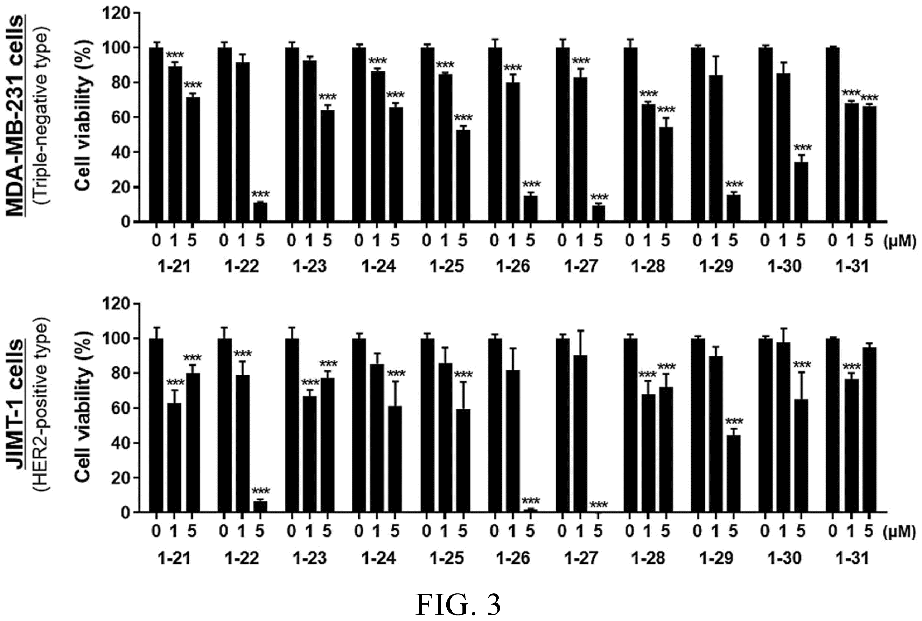

The measured results are shown in FIGS. 1 to 3. As a result, it was confirmed that most of the quinazoline-benzimidazole derivatives of Formulas 1-1 to 1-31 inhibited cell viability in a concentration-dependent manner in the human breast cancer cell lines MDA-MB-231 and JIMT-1.

### 2.2 Confirmation of Apoptosis Effect of Quinazoline-Benzimidazole Derivatives

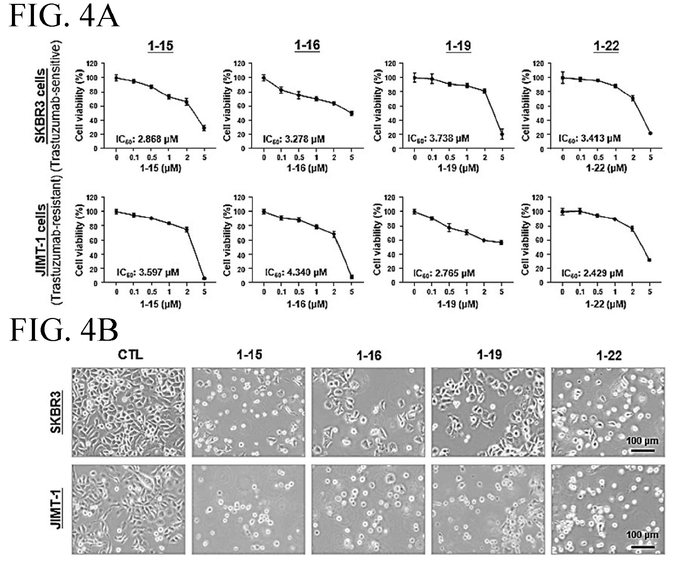

The following experiments were performed with the compounds of Formulas 1-15, 1-16, 1-19, and 1-22, which exhibited excellent activity in Example 2-1 above. After treatment with the above-described compounds at different concentrations (0, 0.1, 0.5, 1, 2, and 5 µM) for 72 hours in a trastuzumab-sensitive HER2-positive breast cancer cell line SKBR3 and a trastuzumab-resistant JIMT-1, concentration-dependent inhibition of cell viability (MTS assay) and IC50 values according to the inhibition of cell viability were confirmed, and the results are shown in A of FIG. 4. In addition, after treatment with compounds 1-15, 1-16, 1-19, and 1-22 at a concentration of 5 µM for 48 hours in SKBR3 and JIMT-1 cell lines, morphological changes of the cells were observed using an optical microscope, and the results are shown in B of FIG. 4. It was confirmed that the breast cancer cell lines exhibited a typical apoptotic morphology by treatment with the quinazoline-benzimidazole derivatives.

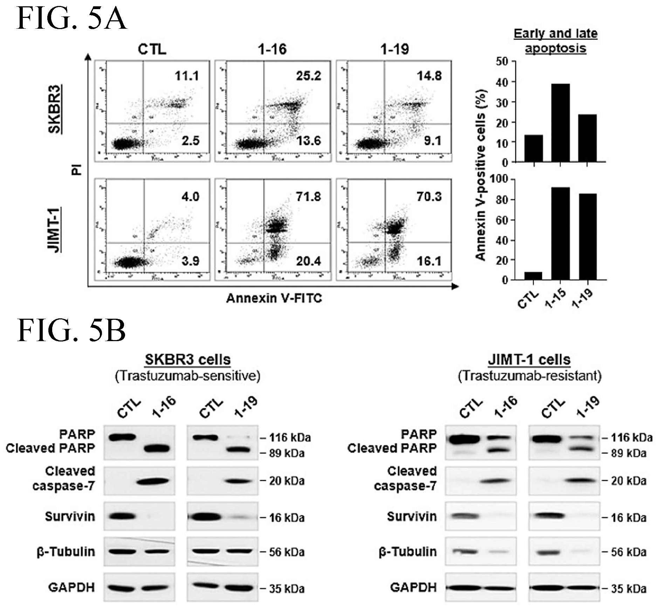

Subsequently, after treatment with the compounds of Formulas 1-16 and 1-19 at a concentration of 5 µM for 48 hours in SKBR3 and JIMT-1 cell lines, the degree of apoptosis was measured by flow cytometry through Annexin V/PI staining, and the results are shown in A of FIG. 5. It was confirmed that the quinazoline-benzimidazole derivatives effectively induced early and late apoptosis in both cell lines. After treatment in the same manner as above, expression of apoptosis-related proteins and degradation of tubulin protein were confirmed by western blot, and the results are shown in B of FIG. 5. Thereby, it was confirmed that the above compounds of the disclosure are capable of exhibiting an apoptosis effect by inhibiting tubulin polymerization.

### 2.3 Confirmation of Mechanism of Action of Quinazoline-Benzimidazole Derivatives

As described above, the quinazoline-benzimidazole derivative of the disclosure is designed such that the quinazoline moiety of the compound confers strong binding to a tyrosine kinase of human epidermal growth factor receptor 2 (HER2), and simultaneously, the benzimidazole moiety binds between tubulin polymers to have an ability to inhibit polymerization.

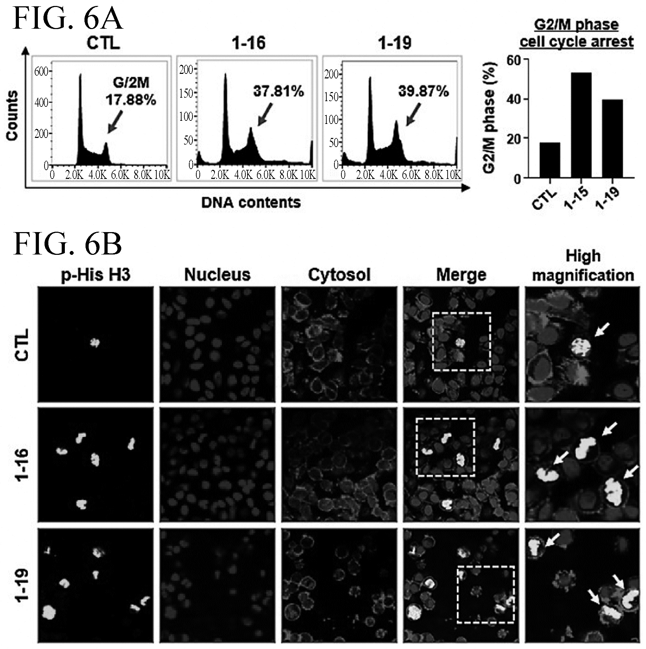

In order to confirm this, the following experiments were performed. After treatment with the compounds of Formulas 1-16 and 1-19 at a concentration of 5 µM for 48 hours in a trastuzumab-resistant HER2-positive breast cancer cell JIMT-1, the cells were stained with propidium iodide (PI), and the cell cycle was analyzed according to DNA content using flow cytometry, and the results are shown in A of FIG. 6. G2/M phase cell cycle arrest by treatment with the quinazoline-benzimidazole derivative was confirmed in JIMT-1.

In addition, after treatment with compounds 1-16 and 1-19 at a concentration of 5 µM for 24 hours in a JIMT-1 cell line, expression of phospho-Histone H3, a marker of mitosis, was confirmed by immunocytochemistry, and the results are shown in B of FIG. 6. It was confirmed that treatment with the quinazoline-benzimidazole derivatives increased abnormal expression of a phospho-Histone H3 protein, and thus, it was confirmed that the quinazoline-benzimidazole derivatives of the disclosure may induce cell cycle arrest through an ability to inhibit tubulin polymerization.

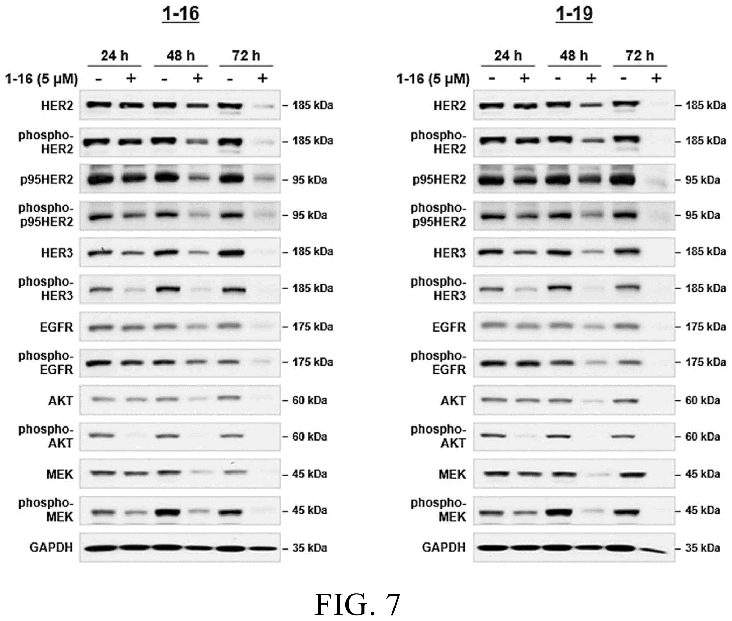

Then, after treatment with the compounds of Formulas 1-16 and 1-19 at a concentration of 5 µM for 24, 48, and 72 hours in a JIMT-1 cell line, protein expression and phosphorylation of HER2, HER family members (HER3 and EGFR), and AKT and MEK of downstream signaling mechanism were confirmed by western blot, and the results are shown in FIG. 7. By treatment with the quinazoline-benzimidazole derivative of the disclosure, expression and phosphorylation of major proteins in the HER2 signaling mechanism were decreased in a time-dependent manner, and in particular, it was confirmed that the activities of HER2, p95HER2, HER3, EGFR, AKT, and MEK, which are major causes of trastuzumab resistance, were effectively inhibited. Thereby, it was confirmed that the quinazoline-benzimidazole derivatives of the disclosure have a tyrosine kinase inhibitory effect on HER2 as in the above-described design objective.

### Example 3. Confirmation of Anticancer Activity of Quinazoline-Benzimidazole Derivatives by Cancer Type

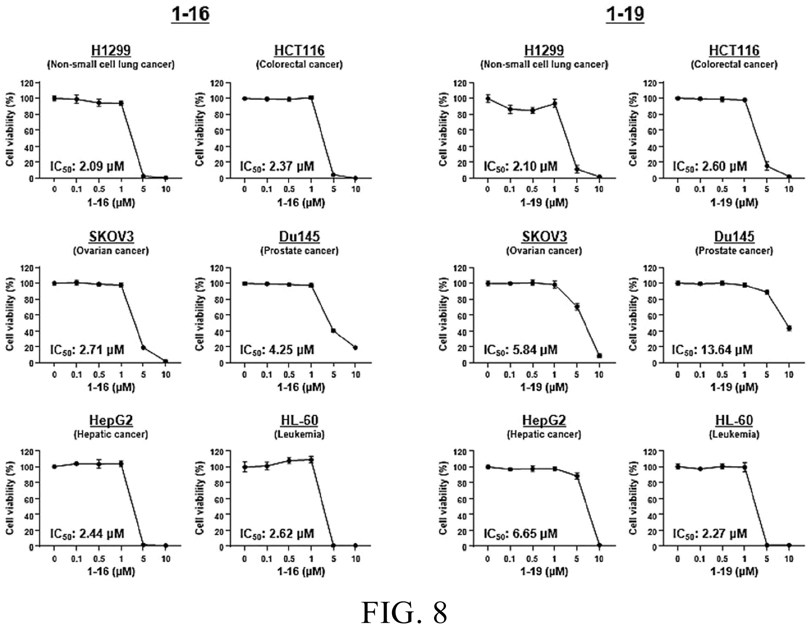

After treatment with quinazoline-benzimidazole derivative compounds 1-16 and 1-19 at different concentrations (0, 0.1, 0.5, 1, 5, and 10 µM) for 72 hours in a lung cancer cell line H1299, a colorectal cancer cell line HCT116, an ovarian cancer cell line SKOV3, a prostate cancer cell line Du145, a hepatic cancer cell line HepG2, and a hematological cancer cell line HL-60, concentration-dependent inhibition of cell viability (MTS assay) and IC50 values according to the inhibition of cell viability were confirmed, and the results are shown in FIG. 8. As shown in the drawings, it was confirmed that cell viability was effectively inhibited in all other cancer-type cell lines by treatment with the quinazoline-benzimidazole derivative of the disclosure.

Although the embodiments are described with reference to the limited drawings, one of ordinary skill in the art may apply various technical modifications and variations based thereon. For example, suitable results may be achieved if the described techniques are performed in a different order, and/or if components in a described system, structure, device, or circuit are combined in a different manner, and/or replaced or supplemented by other components or their equivalents.

Therefore, other implementations, other embodiments, and equivalents to the claims are also within the scope of the following claims.

## Claims

1. A quinazoline-benzimidazole derivative represented by [Formula 1], or a pharmaceutically acceptable salt thereof: 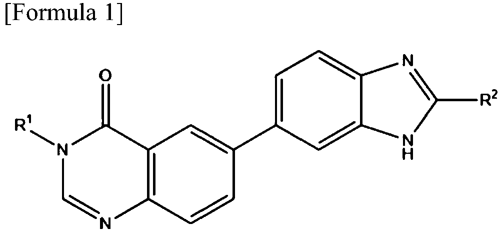 wherein, in Formula 1,
R¹ is -(CH₂)ₙ-A,
A is any one of a substituted or unsubstituted C₃-C₂₀ cycloalkyl and a C₃-C₂₀ heterocycloalkyl,
when the cycloalkyl or the heterocycloalkyl is substituted, the cycloalkyl or the heterocycloalkyl is substituted with one or more selected from the group consisting of a C₁-C₆ alkyl group, a hydroxy group, an amine group, a methoxy group, and a halogen group,
R² is any one selected from the group consisting of a substituted or unsubstituted C₃-C₂₀ aryl, a substituted or unsubstituted C₃-C₂₀ heteroaryl, a substituted or unsubstituted C₃-C₂₀ cycloalkyl, a substituted or unsubstituted C₃-C₂₀ heterocycloalkyl, and a methyl carbamate,
when the aryl, the cycloalkyl, or the heterocycloalkyl is substituted, the aryl, the cycloalkyl, or the heterocycloalkyl is substituted with one or more selected from the group consisting of a C₁-C₆ alkyl group, a halogen group, a hydroxy group, and an alkoxy group, and
n is an integer of 0 to 6.

2. The quinazoline-benzimidazole derivative or the pharmaceutically acceptable salt thereof of claim 1,
wherein, in [Formula 1],
A is a substituted or unsubstituted C₃-C₂₀ heterocycloalkyl,
when the heterocycloalkyl is substituted, the heterocycloalkyl is substituted with one or more of a methyl group and a hydroxy group, R² is any one selected from the group consisting of a substituted or unsubstituted phenyl, a substituted or unsubstituted thiophenyl, a substituted or unsubstituted pyridinyl, a substituted or unsubstituted cyclopentyl, a substituted or unsubstituted cyclohexyl, and -NH-(C=O)-OCH₃,
when the phenyl, the thiophenyl, the pyridinyl, the cyclopentyl, and the cyclohexyl are substituted, the phenyl, the thiophenyl, the pyridinyl, the cyclopentyl, and the cyclohexyl are substituted with one or more selected from the group consisting of methyl, methoxy, halogen, and hydroxy, and n is 2.

3. The quinazoline-benzimidazole derivative or the pharmaceutically acceptable salt thereof of claim 2,
wherein, in [Formula 1],
the C₃-C₂₀ heterocycloalkyl of A is any one of morpholine and piperidine.

4. The quinazoline-benzimidazole derivative or the pharmaceutically acceptable salt thereof of claim 1, wherein the quinazoline-benzimidazole derivative represented by [Formula 1] is any one selected from the group consisting of the following compounds:

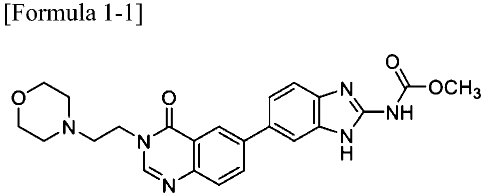

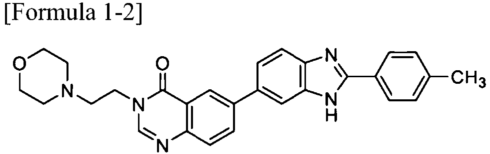

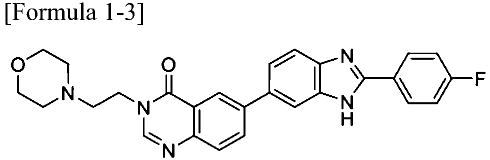

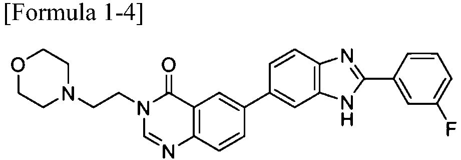

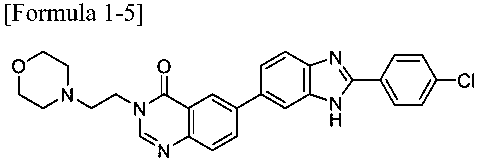

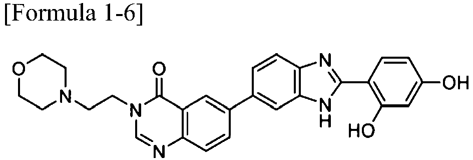

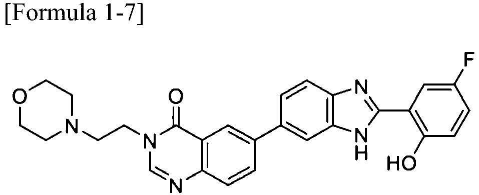

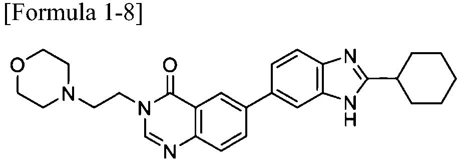

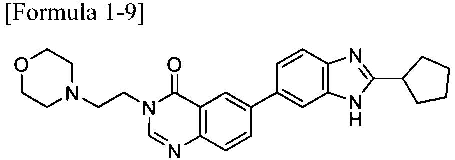

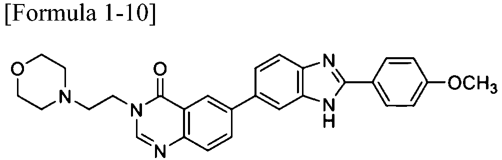

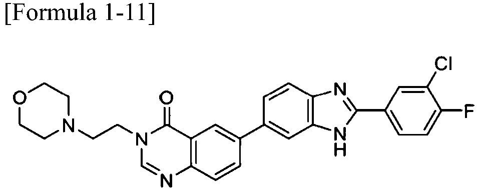

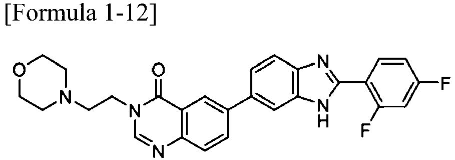

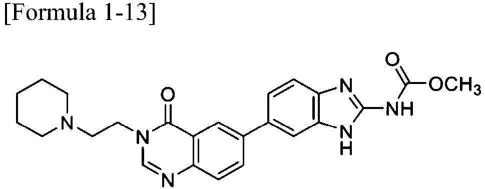

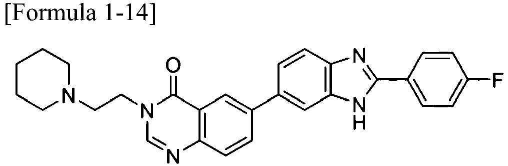

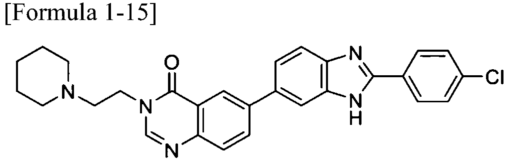

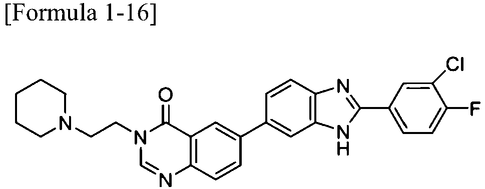

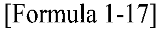

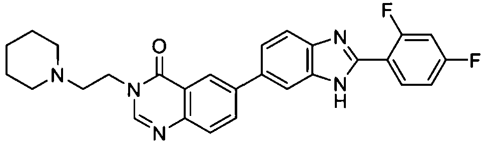

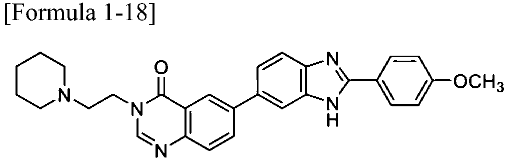

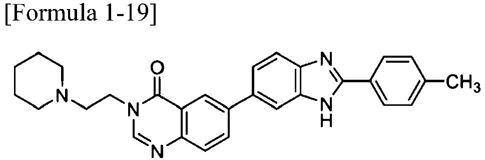

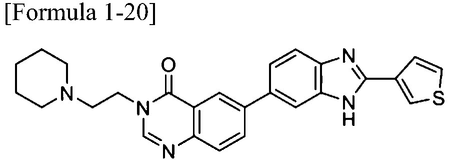

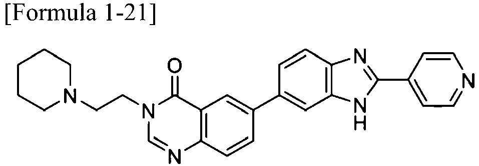

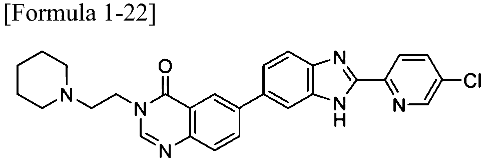

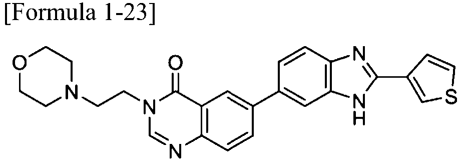

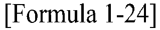

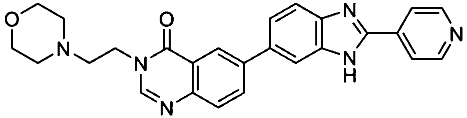

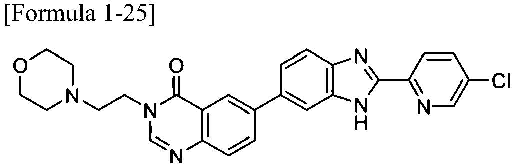

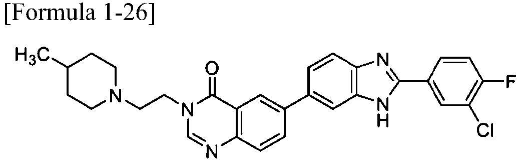

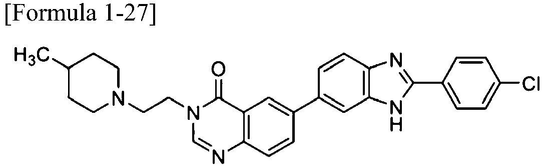

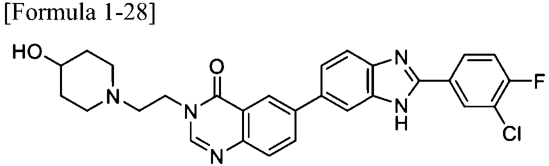

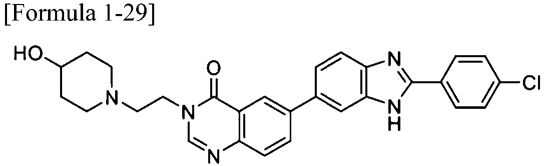

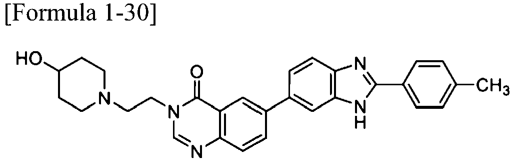

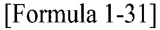

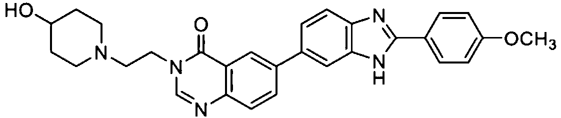

5. The quinazoline-benzimidazole derivative or the pharmaceutically acceptable salt thereof of claim 1, wherein the quinazoline-benzimidazole derivative represented by [Formula 1] is any one selected from the group consisting of the following compounds:

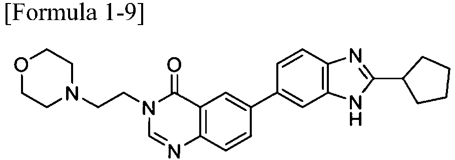

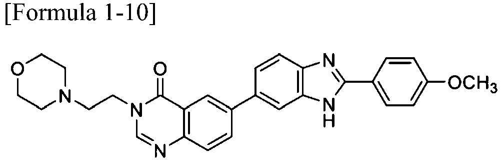

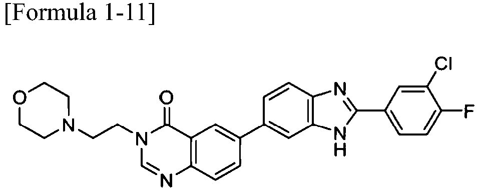

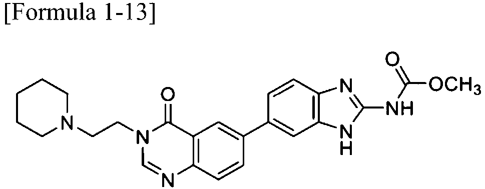

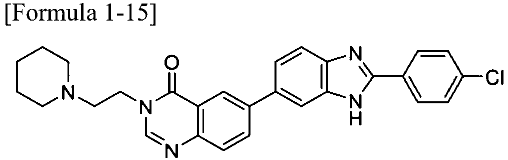

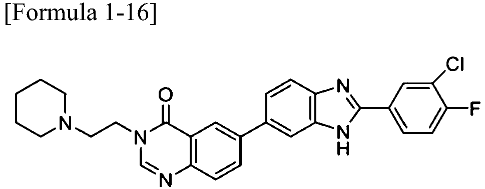

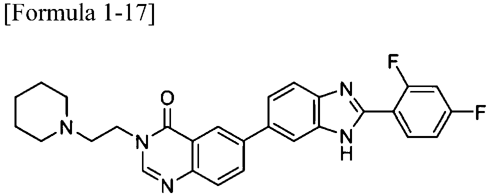

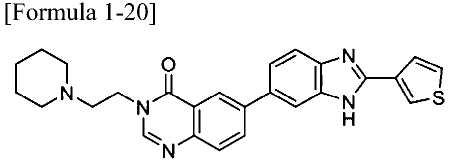

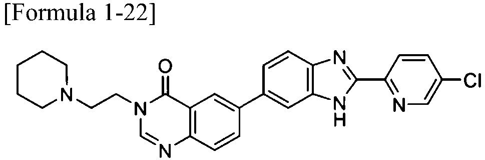

6. The quinazoline-benzimidazole derivative or the pharmaceutically acceptable salt thereof of claim 1, wherein the quinazoline-benzimidazole derivative has both an effect of inhibiting tubulin polymerization and an effect of inhibiting a tyrosine kinase of human epidermal growth factor receptor 2 (HER2).

7. The quinazoline-benzimidazole derivative or the pharmaceutically acceptable salt thereof of claim 1, wherein the pharmaceutically acceptable salt of the quinazoline-benzimidazole derivative is one or more selected from the group consisting of a hydrochloride, a bromate, a sulfate, a phosphate, a nitrate, a citrate, an acetate, a lactate, a tartrate, a maleate, a gluconate, a succinate, a formate, a trifluoroacetate, an oxalate, a fumarate, a glutarate, an adipate, a methanesulfonate, a benzenesulfonate, a para-toluenesulfonate, a camphorsulfonate, a sodium salt, a potassium salt, a lithium salt, a calcium salt, and a magnesium salt.

8. A pharmaceutical composition for preventing or treating cancer, the pharmaceutical composition comprising, as an active ingredient, the quinazoline-benzimidazole derivative of any one of claims 1 to 7, or a pharmaceutically acceptable salt thereof.

9. The pharmaceutical composition of claim 8, wherein the pharmaceutical composition induces apoptosis by causing cell cycle arrest in cancer cells.

10. The pharmaceutical composition of claim 8, wherein the pharmaceutical composition inhibits an activity of one or more of HER2, p95HER2, HER3, EGFR, AKT, and MEK.

11. The pharmaceutical composition of claim 8, wherein the cancer is one or more selected from the group consisting of skin cancer, breast cancer, uterine cancer, esophageal cancer, gastric cancer, brain tumor, colon cancer, rectal cancer, colorectal cancer, lung cancer, ovarian cancer, cervical cancer, endometrial cancer, vulvar cancer, renal cancer, hematological cancer, pancreatic cancer, prostate cancer, testicular cancer, laryngeal cancer, head and neck cancer, thyroid cancer, hepatic cancer, bladder cancer, osteosarcoma, lymphoma, hematological cancer, thymic cancer, urethral cancer, and bronchial cancer.

12. The pharmaceutical composition of claim 8, wherein the cancer is HER2-positive breast cancer or triple-negative breast cancer.

13. A method of preparing a quinazoline-benzimidazole derivative represented by [Formula 1], or a pharmaceutically acceptable salt thereof, the method comprising the following preparation steps:
1) preparing a compound represented by [Formula 4] by reacting a compound represented by R¹-NH₂ with 5-bromoanthranilic acid and triethyl orthoformate;
2) preparing a compound represented by [Formula 2] by reacting the compound represented by [Formula 4] with any one of 2-nitro-4-(4,4,5,5-tetramethyl-1,3,2-dioxaborolan-2-yl)aniline and 4-amino-3-nitrophenylboronic acid pinacol ester;
3) preparing a compound represented by [Formula 3] from the compound represented by [Formula 2]; and
4) preparing a compound represented by [Formula 1] by reacting the compound represented by [Formula 3] with any one of 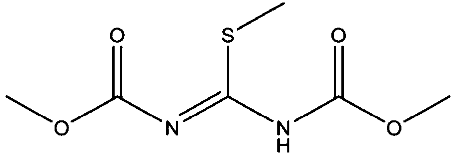 and a compound represented by R²-CHO; 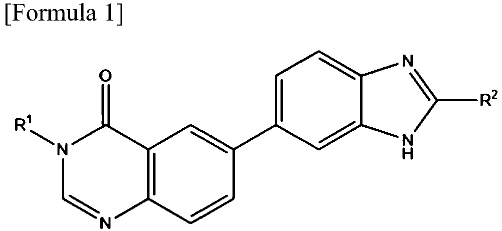 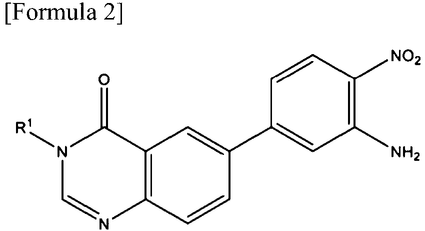 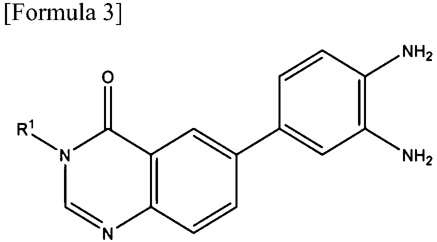 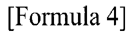 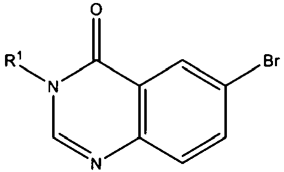 wherein, in Formulas 1 to 4,
R¹ is -(CH₂)ₙ-A,
A is any one of a substituted or unsubstituted C₃-C₂₀ cycloalkyl and a C₃-C₂₀ heterocycloalkyl,
when the cycloalkyl or the heterocycloalkyl is substituted, the cycloalkyl or the heterocycloalkyl is substituted with one or more selected from the group consisting of a C₁-C₆ alkyl group, a hydroxy group, an amine group, a methoxy group, and a halogen group,
R² is any one selected from the group consisting of a substituted or unsubstituted C₃-C₂₀ aryl, a substituted or unsubstituted C₃-C₂₀ heteroaryl, a substituted or unsubstituted C₃-C₂₀ cycloalkyl, a substituted or unsubstituted C₃-C₂₀ heterocycloalkyl, and a methyl carbamate,
when the aryl, the cycloalkyl, or the heterocycloalkyl is substituted, the aryl, the cycloalkyl, or the heterocycloalkyl is substituted with one or more selected from the group consisting of a C₁-C₆ alkyl group, a halogen group, a hydroxy group, and an alkoxy group, and
n is an integer of 0 to 6.
